# EUROPEAN PATENT SPECIFICATION

(11) **EP 4 105 301 B1**
(45) Date of publication and mention of the grant of the patent: **06.11.2024**
(21) Application number: 21179502.6
(22) Date of filing: 15.06.2021
(51) Int. Cl.: C10L 1/2383, C10L 1/198, C10L 1/238, C10L 1/2387, C10L 1/14, C10L 1/224, C10L 10/04, C10L 10/06, C08F 8/00, C08F 8/12, C08F 8/32

(54) **GASOLINE FUEL AND FUEL ADDITIVE PACKAGES COMPRISING AN AMIDE COMPOUND**
MOTORBENZIN UND KRAFTSTOFFADDITIVPAKETE ENTHALTEND EINE AMIDVERBINDUNG
ESSENCE POUR MOTEUR ET PAQUETS D'ADDITIFS COMPRENANT UN AMIDE

(43) Date of publication of application: 21.12.2022
(73) Proprietor: BASF SE, 67056 Ludwigshafen am Rhein (DE)
(72) Inventor: MOLAWI, Kian, 67056 Ludwigshafen (DE); HANSCH, Markus, 67056 Ludwigshafen (DE); WALTER, Marc, 67056 Ludwigshafen (DE); ZORN, Matthias, 67056 Ludwigshafen (DE); MEZGER, Jochen, 67056 Ludwigshafen (DE)
(74) Representative: BASF IP Association

(56) References cited:
- WO-A1-2006/031193
- WO-A1-2009/095443
- WO-A1-2020/006476
- WO-A1-2020/194616
- WO-A1-98/16599
- CN-A- 112 010 773
- FR-A1- 2 764 602
- US-A- 4 832 702
- US-A- 5 496 383
- US-A- 5 567 845

## Description

The present invention describes new gasoline fuels, gasoline additive packagess, and the use of components for gasoline additives.

Reduction of fuel consumption is an ever-existing need in order to increase sustainability of vehicles with combustion engines. One way to reduce fuel consumption is the use of friction modifiers in fuels in order to reduce the friction in the engines.

Chemically speaking, typical friction modifiers are fatty acids, fatty acid amides or esters. However, such friction modifiers show the disadvantage that they tend to form deposits in the engine or exhibit metal leaching. Often such friction modifiers lead to unstable formulations of fuel additive packages or raise formulability issues.

It was an object of the present invention to develop a gasoline fuel with improved fuel efficiency without the disadvantage of the difficult formulability of conventional friction modifiers.

The object was achieved by a gasoline Fuel, comprising at least one amide of formula (I)

R¹-(C=O)-(NR²)-R³

wherein
R¹ is a linear or branched, preferably branched C₇- to C₂₉-alkyl, or C₇- to C₂₉-alkenyl, preferably C₇- to C₂₃-alkyl or C₇- to C₂₃-alkenyl
R² is hydrogen or C₁- to C₄-alkyl, and
R³ is a hydrocarbyl residue comprising 12 to 200 carbon atoms obtainable from polymerisation of an olefin.

The amides of formula (I) do not act as a classical friction modifier but as a fuel economy additive and, therefore, do not exhibit the disadvantages as friction modifiers or to a lesser extent. While a friction modifier reduces wear, preferably measured according to the HFRR-test (High Frequency Reciprocating Rig, CEC F-06-A-96), a fuel economy additive does not or much less than comparable amounts of a friction modifier.

US 7846224 B2 discloses hyper-branched fatty acid amides as friction modifiers with an improved low temperature compatibility in a fuel. In the context of US 7846224 B2 hyper-branched fatty acid are defined as fatty acids with at least two substituents on the alpha-carbon.

The only fatty acid explicitly disclosed in the examples is isostearic acid, no effect on fuel consumption is disclosed.

EP 1252267 B1 discloses fatty acids having 10-30 carbon atoms derived from naturally occurring fats and oils and alkylamine salts, alkylamides and alkylesters thereof as friction reducing additives.

Only amine component disclosed is n-butyl amine, amides are not explicitly disclosed.

The amides of formula (I) are obtainable, preferably obtained from carboxylic acids R¹-COOH or derivatives thereof with amines R³-NHR², wherein R¹, R², and R³ are defined below.

Derivatives of carboxylic acids are
- their alkyl esters, preferably C₁-to C₄-alkylesters, more preferably methyl- or ethyl esters, and most preferably methyl esters,
- their anhydrides or
- their chlorides.

Preferred derivatives of carboxylic acids are alkyl esters.

Substituents R¹ are C₇- to C₂₉-alkyl or C₇- to C₂₉-alkenyl, preferably C₇- to C₂₃-alkyl or C₇- to C₂₃-alkenyl, more preferably C₇- to C₁₇-alkyl or C₇- to C₁₇-alkenyl.

Examples for carboxylic acids R¹-COOH are octanoic acid (caprylic acid), 2-ethylhexanoic acid, 3,5,5-trimethyl hexanoic acid, nonanoic acid, isononanoic acid, 2-propylheptanoic acid, decanoic acid (capric acid), undecanoic acid, dodecanoic acid (lauric acid), tridecanoic acid, tetradecanoic acid (myristic acid), hexadecanoic acid (palmitic acid), heptadecanoic acid, octadecanoic acid (stearic acid), isostearic acid, oleic acid, linoleic acid, linolaidic acid, erucic acid, arachidic acid, behenic acid, lignoceric acid and cerotic acid. The above monocarboxylic acids, including the so called fatty acids, may be of synthetic or of natural origin. Mixtures of the above aliphatic monocarboxylic acids can also be used.

In one embodiment of the present invention the moiety R¹ is a branched alkyl residue.

In a preferred embodiment the carboxylic acid R¹-COOH is isononanoic acid.

As used herein, isononanoic acid refers to one or more branched-chain aliphatic carboxylic acids with 9 carbon atoms. Embodiments of isononanoic acid may include 7-methyloctanoic acid (e.g., CAS Nos. 693-19-6 and 26896-18-4), 6,6-dimethylheptanoic acid (e.g., CAS No. 15898-92-7), 3,5,5-trimethylhexanoic acid (e.g., CAS No. 3302-10-1), 3,4,5-trimethylhexanoic acid, 2,5,5-trimethylhexanoic acid, 2,2,4,4-tetramethylpentanoic acid (e.g., CAS No. 3302-12-3) and combinations thereof. In a preferred embodiment, isononanoic acid has as its main component greater than 90% of one of 7-methyloctanoic acid, 6,6-dimethylheptanoic acid, 3,5,5-trimethylhexanoic acid, 3,4,5-trimethylhexanoic acid, 2,5,5-trimethylhexanoic acid, and 2,2,4,4-tetramethylpentanoic acid. The balance of the isononanoic acid may include other nine carbon carboxylic acid isomers and minor amounts of one or more contaminants. In a preferred embodiment, the isononanoic acid has as its main component greater than 90% of 3,5,5-trimethylhexanoic acid and even more preferably, the main component is greater than 95% 3,5,5-trimethylhexanoic acid.

In another embodiment the carboxylic acid R¹-COOH is a fatty acid or a mixture of fatty acids comprising from 8 to 18 carbon atoms, preferably from 10 to 16 carbon atoms.

Such mixtures of fatty acids may be derived from natural sources, e.g. from vegetable oils such as castor oil, olive oil, peanut oil, palm kernel oil, coconut oil, mustard oil, cottonseed oil, and especially sunflower oil, palm oil, soybean oil and rapeseed oil. Further examples include oils which can be obtained from wheat, jute, sesame and shea tree nut; it is additionally also possible to use arachis oil, jatropha oil and linseed oil. The extraction of these oils and the conversion thereof to the fatty acids are known from the prior art or can be inferred therefrom.

Especially such mixtures of fatty acids encompass caprylic acid, capric acid, lauric acid, myristic acid, palmitic acid, oleic acid, stearic acid, elaidic acid, and linoleic acid or mixtures thereof.

In the amines R³-NHR² the residue R² is hydrogen or C₁- to C₄-alkyl, preferably hydrogen or methyl, and more preferably hydrogen.

The residue R³ is a hydrocarbyl residue comprising 12 to 200, preferably 16 to 150, more preferably 20 to 100, even more preferably 30 to 90, and especially 40 to 80 carbon atoms obtainable from polymerisation of an olefin, preferably from polymerisation of olefin mixtures comprising propene, 1-butene or iso-butene, more preferably from propene or iso-butene, and most preferably from iso-butene.

In one embodiment of the invention, the polyalkenes as basis for the residue R³ used for the preparation are derived from olefin polymers. The olefin polymers may comprise homopolymers and co-polymers of polymerizable olefin monomers having 2 to about 16 carbon atoms, 2 to about 6 carbon atoms or 2 to about 4 carbon atoms.

Interpolymers are those in which two or more olefin monomers are interpolymerized by known conventional methods, giving polyalkenes having units derived from each of the two or more olefin monomers within their structure. Thus, "interpolymers" comprise copolymers, terpolymers and tetrapolymers.

"Polyalkenes", from which the polyalkene-substituted amines are derived, are conventionally frequently also referred to as "polyolefins".

The olefin monomers from which the olefin polymers are derived are polymerizable olefin monomers having one or more ethylenically unsaturated groups (i.e. >C=C<); in other words, they are monoolefinic monomers such as ethylene, propylene, 1-butene, isobutene (2-methyl-1-butene), 1-octene, or polyolefinic monomers (usually diolefinic monomers) such as 1,3-butadiene and isoprene.

The olefin monomers are usually polymerizable terminal olefins, i.e. olefins having the >C=CH2 group in their structure. However, it is also possible to use polymerizable internal olefin monomers characterized by groups of the formula >C-C=C-C<.

Specific examples of terminal and internal olefin monomers which can be used to prepare the polyalkenes by conventional methods are: ethylene, propylene, the butenes (butylene), especially 1-butene, 2-butene and isobutylene, 1-pentene, 1-hexene, 1-heptene, 1-octene, 1-nonene, 1-decene, 2-pentene, propylene tetramer, diisobutylene, isobutylene trimer, 1,2-butadiene, 1,3-butadiene, 1,2-pentadiene, 1,3-pentadiene, 1,4-pentadiene, isoprene, 1,5-hexadiene, 2-methyl-5-propyl-1-hexene, 3-pentene, 4-octene and 3,3-dimethyl-1-pentene.

In another embodiment, the olefin polymer is preparable by polymerization of a C4 refinery stream having a butene content of about 35 to about 75 percent by weight and an isobutene content of about 30 to about 60 percent by weight in the presence of a Lewis acid catalyst such as aluminum trichloride or boron trifluoride. These polybutenes typically comprise predominantly (more than about 80% of all the repeat units) repeat isobutene units of the (-CH₂-C(CH₃)₂-) type.

In a further embodiment, the polyalkene substituent of the polyalkene-substituted amine is derived from a polyisobutylene.

In a preferred embodiment the amines R³-NHR² are obtainable by polymerisation of the above-mentioned olefins, followed by hydroformylation and reductive amination, preferably with ammonia.

Such a process for preparing a polyalkene-substituted amine comprises the reaction of a hydro-formylated olefin with a polyamine and hydrogenation of the reaction product is described in US 5,567,845 and 5,496,383.

Another process for preparing a polyalkene-substituted amine comprises hydroformylation of a polybutene or polyisobutylene with a catalyst, such as rhodium or cobalt, in the presence of CO and hydrogen at elevated pressures and temperatures, as described in US 4,832,702.

In another, however less preferred embodiment the amines R³-NHR² with R² being hydrogen are obtainable by a process according to which polyisobutene with a high double bond content is reacted with nitrogen oxides or mixtures of nitrogen oxides and oxygen (WO 96/03367 A1). Subsequent elimination and hydrogenation yields polyisobutene amines which comprise one carbon atom less than the polyisobutene amines according to the before-mentioned manufacturing process, since the hydroformylation step is omitted.

A further process for preparing a polyalkene-substituted amine comprises the conversion of a polyalkene with the aid of a conventional epoxidizing reagent with or without catalyst to the corresponding epoxide and the conversion of the epoxide to the polyalkene-substituted amine by reaction with ammonia or an amine under the conditions of reductive amination, as described in US 5,350,429.

A process for preparing a polyalkene-substituted amine comprises the reaction of a halogenated olefin polymer with an amine, as described in US Patents 3,275,554, 3,438,757, 3,454,555, 3,565,804, 3,755,433 and 3,822,289.

A further process for preparing a polyalkene-substituted amine comprises the hydrogenation of a β-amino nitrile which has been prepared by reaction of an amine with a nitrile, as described in US 5,492,641.

Preferably in the amine R³-NHR² the residue R² is hydrogen and the residue R³ is derived from a polyisobutene with the number average molecular weight Mn of from 168 to 2300, more preferably of from 224 to 1500, even more preferably of from 550 to 1300, most preferably of from 700 to 1300, and especially of 950 to 1050.

In a preferred embodiment these amines are obtainable by oligomerization of propene, isobutene, 1-butene or 2-butene, preferably propene or isobutene, especially isobutene forming a double bond-containing oligomer mixture or polymer, followed by hydroformylation and reductive amination with ammonia.

Preferably, the oligomers or polymers comprising isobutene in copolymerized form have a high content of terminal ethylenic double bonds (a-double bonds), for example at least 50 mol%, preferably at least 60 mol%, more preferably at least 70 mol% and most preferably at least 80 mol%.

For the preparation of such oligomers or polymers comprising isobutene in copolymerized form, suitable isobutene sources are either pure isobutene or isobutene-containing C4 hydrocarbon streams, for example C4 raffinates, especially "raffinate 1", C4 cuts from isobutane dehydrogenation, C4 cuts from steamcrackers and from FCC crackers (fluid catalyzed cracking), provided that they have substantially been freed of 1,3-butadiene present therein. A C4 hydrocarbon stream from an FCC refinery unit is also known as a "b/b" stream. Further suitable isobutene-containing C4 hydrocarbon streams are, for example, the product stream of a propylene-isobutane cooxidation or the product stream from a metathesis unit, which are generally used after customary purification and/or concentration. Suitable C4 hydrocarbon streams comprise generally less than 500 ppm, preferably less than 200 ppm, of butadiene. The presence of 1-butene and of cis- and trans-2-butene is substantially uncritical. Typically, the isobutene concentration in said C4 hydrocarbon streams is in the range from 40% to 60% by weight. For instance, raffinate 1 generally consists essentially of 30% to 50% by weight of isobutene, 10% to 50% by weight of 1-butene, 10% to 40% by weight of cis- and trans-2-butene and 2% to 35% by weight of butanes; in the polymerization process the unbranched butenes in the raffinate 1 are generally virtually inert, and only the isobutene is polymerized.

In a preferred embodiment, the monomer source used for polymerization is a technical C4 hydrocarbon stream having an isobutene content of 1% to 100% by weight, especially of 1% to 99% by weight, in particular of 1% to 90% by weight, more preferably of 30% to 60% by weight, especially a raffinate 1 stream, a b/b stream from an FCC refinery unit, a product stream from a propylene-isobutane cooxidation or a product stream from a metathesis unit.

Especially when a raffinate 1 stream is used as isobutene source, the use of water as the sole initiator or as further initiator has been found to be useful, particularly when polymerization is effected at temperatures of -20°C to +30°C, especially of 0°C to +20°C. At temperatures of - 20°C to +30°C, especially of 0°C to +20°C, however, it is possible to dispense with the use of an initiator when using a raffinate 1 stream as isobutene source.

Said isobutene-containing monomer mixture may comprise small amounts of contaminants such as water, carboxylic acids or mineral acids without causing any critical yield or selectivity losses. It is appropriate to the purpose to avoid accumulation of these impurities by removing such harmful substances from the isobutene-containing monomer mixture, for example, by adsorption on solid adsorbents such as activated carbon, molecular sieves or ion exchangers.

It is also possible, albeit less preferable, to convert monomer mixtures of isobutene or of the isobutene-containing hydrocarbon mixture with olefinically unsaturated monomers copolymerizable with isobutene. If monomer mixtures of isobutene with suitable comonomers are to be copolymerized, the monomer mixture comprises preferably at least 5% by weight, more preferably at least 10% by weight and especially at least 20% by weight of isobutene, and preferably at most 95% by weight, more preferably at most 90% by weight and especially at most 80% by weight of comonomers.

Due to their high proportion of vinylidene double bonds these polyisobutenes are especially reactive to undergo hydroformylation and subsequent amination, preferably with ammonia, to yield the corresponding polyisobutene amines as amines R³-NHR², which represents a preferred embodiment of the present invention.

Such polyisobutene amines are commercially available from BASF SE, Ludwigshafen, under the tradename KEROCOM(R) PIBA.

The amides according to formula (I) can be prepared by reacting an amine R³-NHR² with a carboxylic acid R¹-COOH or its derivatives.

Preferably the carboxylic acid R¹-COOH or its alkyl ester, more preferably the carboxylic acid or its methyl- or ethyl ester, even more preferably the carboxylic acid or its methyl ester, and especially the free carboxylic acid R¹-COOH is used.

In a preferred embodiment amine R³-NHR² and carboxylic acid R¹-COOH or its derivatives are mixed in a stoichiometric ratio of 2 : 1 to 1 : 2, preferably 1.5 : 1 to 1 : 1.5, more preferably 1.2 : 1 to 1 : 1.2, most preferably 1.1 : 1 to 1 : 1.1, and especially 1 : 1 and heated to a temperature from 130 to 220 °C, preferably 140 to 200 °C, more preferably 150 to 180 °C for a period of 1 to 24 hours, preferably 3 to 22, more preferably 5 to 20 hours.

The reaction may be conducted neat or preferably in a solvent. Suitable solvents are, for example, nonpolar organic solvents such as aromatic and aliphatic hydrocarbons, for example toluene, xylenes, white spirit and products sold under the trade names SHELLSOL (Royal Dutch/Shell Group), Solvesso, and EXXSOL (ExxonMobil). If a solvent is used its boiling temperature or boiling range should be chosen above the reaction temperature. As an alternative, however less preferred, the reaction may be conducted under pressure, e.g. at a pressure of up to 20 bar, preferably up to 10 bar, more preferably up to 5 bar, even more preferably up to 2 bar. The removal of volatile compounds or reaction products can preferably be supported by applying vacuo, e.g. down to 500 mbar, preferably 200, more preferably 100, even more preferably 50, and especially down to 20 mbar.

In a preferred embodiment the reaction is conducted under atmospheric pressure or under vacuo, more preferably at the beginning the reaction is started under atmospheric pressure and the pressure is reduced continuously or stepwise while the reaction advances.

Usually the reaction is conducted until the calculated amount of water, if a free carboxylic acid is used, or alcohol, if an ester is used, is formed.

Often a turnover of the reaction compounds, especially of the under-stoichiometric compound in the reaction mixture of at least 90% is sufficient, preferably of at least 95%, more preferably of at least 98%.

Due to incomplete reaction the reaction medium may exhibit an acid number of up to 10 mg KOH/g determined according to DIN 53240-1, preferably up to 8 mg KOH/g, more preferably up to 5 mg KOH/g, most preferably up to 3 mg KOH/g, and especially up to 2 mg KOH/g.

The amine number of the reaction mixture according to DIN 16945: 1989-03 (titration with perchloric acid) may be up to 15 mg KOH/g, preferably up to 10 mg KOH/g, more preferably up to 8 mg KOH/g, most preferably up to 5 mg KOH/g, and especially up to 3 mg KOH/g.

Amounts of unreacted carboxylic acid or amine in the reaction mixture may be removed by washing with acidic and basic washing water. Preferably unreacted carboxylic acid and amine remains in the reaction mixture and may even lead to an advantageous effect since carboxylic acid R¹-COOH often exhibits an anticorrosive effect and the amine R³-NHR² acts as a deposit control agent (see below).

If a solvent is used, it is also preferably left in the reaction mixture.

The object of the present invention are gasoline fuels, comprising at least one amide of formula (I) which are used in the gasoline fuels in amounts of from 50 to 1000 ppm by weight, preferably from 75 to 750 ppm by weight, more preferably from 100 to 500 ppm by weight.

The amides of formula (I) may be used as additives or in additive packages in gasoline fuels for improving the fuel efficiency and/or as a a fuel economy additive and/or for reducing or removing intake valve deposits, and/or reducing or removing the fouling of injector nozzles, especially in direct injection spark ignition engines.

It is a further advantage that the amount of friction modifier usually present in additive packages may be reduced: Since the effect of a friction modifier is to reduce wear and to reduce fuel consumption, the latter effect is now achieved by the amides of formula (I), hence, the amount of friction modifier may be reduced or the friction modifier may even be substituted by the amides according to formula (I).

Another advantage of the amides according to formula (I) is that they act as deposit control additives, hence, the amount other deposit control additives (see below) may be reduced.

Therefore, it is an object to use the amides of formula (I) as additives in gasoline fuels, preferably for achieving one or more of the mentioned advantages.

Another object of the present invention are fuel additive packages comprising at least one amide of formula (I), further comprising at least one deposit control agent, selected from the group consisting of
- quaternary ammonium compounds,
- Mannich adducts, and
- polyalkenemono- or polyalkenepolyamines having a number average molecular weight in the range 300 to 5000,
   preferably selected from the group consisting of
- quaternary ammonium compounds, and
- polyalkenemono- or polyalkenepolyamines having a number average molecular weight in the range 300 to 5000.

The gasoline additives may comprise further gasoline additives selected from the group consisting of carrier oils, corrosion inhibitors, and solvent.

Most preferably the deposit control agent is a polyalkenemono- or polyalkenepolyamines, especially a polyisobutene amine having a number average molecular weight in the range 300 to 5000.

The deposit control agents are described in more detail below:

### Quaternary ammonium compounds

The at least one quaternary nitrogen component refer, in the context of the present invention, to nitrogen compounds quaternized in the presence of an acid or in an acid-free manner, preferably obtainable by addition of a compound comprising at least one oxygen- or nitrogen-containing group reactive with an anhydride and additionally at least one quaternizable amino group onto a polycarboxylic anhydride compound and subsequent quaternization.

In most cases the quaternary nitrogen component is an ammonium compound, however in the context of the present document morpholinium, piperidinium, piperazinium, pyrrolidinium, imidazolinium or pyridinium cations are also encompassed by the phrase "quaternary nitrogen component".

The quaternary ammonium compounds are preferably of the formula

⁺NR⁴R⁵R⁶R⁷ A⁻

in which
A⁻ stands for an anion, preferably a carboxylate R⁸COO⁻ or a carbonate R⁸O-COO⁻,
   and
R⁴, R⁵, R⁶, R⁷, and R⁸ independently of another are an organic residue with from 1 to 100 carbon atoms, substituted or unsubstituted, preferably unsubstituted, linear or branched alkyl, alkenyl or hydroxyalkyl residue with 1 to 100, more preferably 1 to 75, even more preferably 1 to 30, most preferably 1 to 25 and especially 1 to 20 carbon atoms,

R⁸ additionally may be substituted or unsubstituted cycloalkyl or aryl residues bearing 5 to 20, preferably 5 to 12 carbon atoms.

It is also possible that the anion may be multiply charged negatively, e.g. if anions of dibasic acids are used, in this case the stoichiometric ratio of the ammonium ions to the anions corresponds to the ratio of positive and negative charges.

The same is true for salts in which the cation bears more than one ammonium ion, e.g. of the substituents connect two or more ammonium ions.

In the organic residues the carbon atoms may be interrupted by one or more oxygen and/or sulphur atoms and/or one or more substituted or unsubstituted imino groups, and may be substituted by C₆-C₁₂-aryl, C₅-C₁₂-cycloalkyl or a five- or six-membered, oxygen-, nitrogen- and/or sulphur-containing heterocycle or two of them together form an unsaturated, saturated or aromatic ring which may be interrupted by one or more oxygen and/or sulphur atoms and/or one or more substituted or unsubstituted imino groups, where the radicals mentioned may each be substituted by functional groups, aryl, alkyl, aryloxy, alkyloxy, halogen, heteroatoms and/or heterocycles.

Two of the residues R⁴ to R⁷ may together form an unsaturated, saturated or aromatic ring, preferably a five-, six- or seven-membered ring (including the nitrogen atom of the ammonium ion).

In this case the ammonium cation may be a morpholinium, piperidinium, piperazinium, pyrrolidinium, imidazolinium or pyridinium cation.

In these definitions
C₁-C₂₀-alkyl which may be substituted by functional groups, aryl, alkyl, aryloxy, alkyloxy, halogen, heteroatoms and/or heterocycles is, for example, methyl, ethyl, propyl, isopropyl, n-butyl, sec-butyl, tert-butyl, pentyl, hexyl, heptyl, octyl, 2-ethylhexyl, 2,4,4-trimethylpentyl, decyl, dodecyl, tetradecyl, heptadecyl, octadecyl, eicosyl, 1,1-dimethylpropyl, 1,1-dimethylbutyl, 1,1,3,3-tetramethylbutyl, benzyl, 1-phenylethyl, 2-phenylethyl, α,α-dimethylbenzyl, benzhydryl, p-tolylmethyl,1-(p-butylphenyl)ethyl, p-chlorobenzyl, 2,4-dichlorobenzyl, p-methoxybenzyl, methoxybenzyl, 2-cyanoethyl, 2-cyanopropyl, 2-methoxycarbonylethyl, 2-ethoxycarbonylethyl, 2-butoxycarbonylpropyl, 1,2-di-(methoxycarbonyl)ethyl, 2-methoxyethyl, 2-ethoxyethyl, 2-butoxyethyl, diethoxymethyl, diethoxyethyl, 1,3-dioxolan-2-yl, 1,3-dioxan-2-yl, 2-methyl-1,3-dioxolan-2-yl, 4-methyl-1,3-dioxolan-2-yl, 2-isopropoxyethyl, 2-butoxypropyl, 2-octyloxyethyl, chloromethyl, 2-chloroethyl, trichloromethyl, trifluoromethyl, 1,1-dimethyl-2-chloroethyl, 2-methoxyisopropyl, 2-ethoxyethyl, butylthiomethyl, 2-dodecylthioethyl, 2-phenylthioethyl, 2,2,2-trifluoroethyl, 2-hydroxyethyl, 2-hydroxypropyl, 3-hydroxypropyl, 4-hydroxybutyl, 6-hydroxyhexyl, 2-aminoethyl, 2-aminopropyl, 3-aminopropyl, 4-aminobutyl, 6-aminohexyl, 2-methylaminoethyl, 2-methylaminopropyl, 3-methylaminopropyl, 4-methylaminobutyl, 6-methylaminohexyl, 2-dimethylaminoethyl, 2-dimethylaminopropyl, 3-dimethylaminopropyl, 4-dimethylaminobutyl, 6-dimethylaminohexyl, 2-hydroxy-2,2-dimethylethyl, 2-phenoxyethyl, 2-phenoxypropyl, 3-phenoxypropyl, 4-phenoxybutyl, 6-phenoxyhexyl, 2-methoxyethyl, 2-methoxypropyl, 3-methoxypropyl, 4-methoxybutyl, 6-methoxyhexyl, 2-ethoxyethyl, 2-ethoxypropyl, 3-ethoxypropyl, 4-ethoxybutyl or 6-ethoxyhexyl, and
C₂-C₂₀-alkyl interrupted by one or more oxygen and/or sulphur atoms and/or one or more substituted or unsubstituted imino groups is, for example, 5-hydroxy-3-oxa-pentyl, 8-hydroxy-3,6-dioxaoctyl, 11-hydroxy-3,6,9-trioxaundecyl, 7-hydroxy-4-oxaheptyl, 11-hydroxy-4,8-dioxaundecyl, 15-hydroxy-4,8,12-trioxapentadecyl, 9-hydroxy-5-oxanonyl, 14-hydroxy-5,10-oxatetradecyl, 5-methoxy-3-oxapentyl, 8-methoxy-3,6-dioxaoctyl, 11-methoxy-3,6,9-trioxaundecyl, 7-methoxy-4-oxaheptyl, 11-methoxy-4,8-dioxa-undecyl, 15-methoxy-4,8,12-trioxapentadecyl, 9-methoxy-5-oxanonyl, 14-methoxy-5,10-oxatetradecyl, 5-ethoxy-3-oxapentyl, 8-ethoxy-3,6-dioxaoctyl, 11-ethoxy-3,6,9-trioxaundecyl, 7-ethoxy-4-oxaheptyl, 11-ethoxy-4,8-dioxaundecyl, 15-ethoxy-4,8,12-trioxapentadecyl, 9-ethoxy-5-oxanonyl or 14-ethoxy-5,10-oxatetradecyl.

If two radicals form a ring, they can together be 1,3-propylene, 1,4-butylene, 1,5-pentylene, 2-oxa-1,3-propylene, 1-oxa-1 ,3-propylene, 2-oxa-1,3-propylene, 1-oxa-1,3-propenylene, 1-aza-1,3-propenylene, 1-C₁-C₄-alkyl-1-aza-1,3-propenylene, 1,4-buta-1,3-dienylene, 1-aza-1,4-buta-1,3-dienylene or 2-aza-1,4-buta-1,3-dienylene.

The number of oxygen and/or sulphur atoms and/or imino groups is not subject to any restrictions. In general, there will be no more than 5 in the radical, preferably no more than 4 and very particularly preferably no more than 3.

Furthermore, there is generally at least one carbon atom, preferably at least two carbon atoms, between any two heteroatoms.

Substituted and unsubstituted imino groups can be, for example, imino, methylimino, isopropylimino, n-butylimino or tert-butylimino.

Furthermore,
functional groups can be carboxy, carboxamide, hydroxy, di(C₁-C₄-alkyl)amino, C₁-C₄-alkyloxycarbonyl, cyano or C₁-C₄-alkyloxy,
C₆-C₁₂-aryl which may be substituted by functional groups, aryl, alkyl, aryloxy, alkyloxy, halogen, heteroatoms and/or heterocycles is, for example, phenyl, tolyl, xylyl, α-naphthyl, β-naphthyl, 4-diphenylyl, chlorophenyl, dichlorophenyl, trichlorophenyl, difluorophenyl, methylphenyl, dimethylphenyl, trimethylphenyl, ethylphenyl, diethylphenyl, isopropylphenyl, tert-butylphenyl, dodecylphenyl, methoxyphenyl, dimethoxyphenyl, ethoxyphenyl, hexyloxyphenyl, methylnaphthyl, isopropylnaphthyl, chloronaphthyl, ethoxynaphthyl, 2,6-dimethylphenyl, 2,4,6-trimethylphenyl, 2,6-dimethoxyphenyl, 2,6-dichlorophenyl, 4-bromophenyl, 2- or 4-nitrophenyl, 2,4- or 2,6-dinitrophenyl, 4-dimethylaminophenyl, 4-acetylphenyl, methoxyethylphenyl or ethoxymethylphenyl,
C₅-C₁₂-cycloalkyl which may be substituted by functional groups, aryl, alkyl, aryloxy, alkyloxy, halogen, heteroatoms and/or heterocycles is, for example, cyclopentyl, cyclohexyl, cyclooctyl, cyclododecyl, methylcyclopentyl, dimethylcyclopentyl, methylcyclohexyl, dimethylcyclohexyl, diethylcyclohexyl, butylcyclohexyl, methoxycyclohexyl, dimethoxycyclohexyl, diethoxycyclohexyl, butylthiocyclohexyl, chlorocyclohexyl, dichlorocyclohexyl, dichlorocyclopentyl or a saturated or unsaturated bicyclic system such as norbornyl or norbornenyl,
a five- or six-membered, oxygen-, nitrogen- and/or sulphur-containing heterocycle is, for example, furyl, thienyl, pyrryl, pyridyl, indolyl, benzoxazolyl, dioxolyl, dioxyl, benzimidazolyl, benzothiazolyl, dimethylpyridyl, methylquinolyl, dimethylpyrryl, methoxyfuryl, dimethoxypyridyl, difluoropyridyl, methylthienyl, isopropylthienyl or tert-butylthienyl and
C₁ to C₄-alkyl is, for example, methyl, ethyl, propyl, isopropyl, n-butyl, sec-butyl or tert-butyl.

The residues R⁴ to R⁸ are preferably C₂-C₁₈-alkyl or C₆-C₁₂-aryl, more preferably C₄-C₁₆-alkyl or C₆-C₁₂-aryl, and even more preferably C₄-C₁₆-alkyl or C₆-aryl.

The residues R⁴ to R⁸ may be saturated or unsaturated, preferably saturated.

Preferred residues R⁴ to R⁸ do not bear any heteroatoms other than carbon or hydrogen.

Preferred examples of R⁴ to R⁷ are methyl, ethyl, propyl, isopropyl, n-butyl, sec-butyl, tert-butyl, pentyl, hexyl, heptyl, octyl, 2-ethylhexyl, 2,4,4-trimethylpentyl, 2-propylheptyl, decyl, dodecyl, tetradecyl, heptadecyl, octadecyl, eicosyl, 1,1-dimethylpropyl, 1,1-dimethylbutyl, 1,1,3,3-tetramethylbutyl, benzyl, 1-phenylethyl, 2-phenylethyl, α,α-dimethylbenzyl, benzhydryl, p-tolylmethyl or 1-(p-butylphenyl)ethyl.

In a preferred embodiment at least one of the residues R⁴ to R⁷ is selected from the group consisting of 2-hydroxyethyl, hydroxyprop-1-yl, hydroxyprop-2-yl, 2-hydroxybutyl or 2-hydroxy-2-phenylethyl.

In one embodiment R⁸ is a polyolefin-homo- or copolymer, preferably a polypropylene, polybutene or polyisobutene residue, with a number-average molecular weight (Mₙ) of 85 to 20000, for example 113 to 10 000, or 200 to 10000 or 350 to 5000, for example 350 to 3000, 500 to 2500, 700 to 2500, or 800 to 1500. Preferred are polypropenyl, polybutenyl and polyisobutenyl radicals, for example with a number-average molecular weight Mₙ of 3500 to 5000, 350 to 3000, 500 to 2500, 700 to 2500 and 800 to 1500 g/mol.

Preferred examples of anions A⁻ are the anions of acetic acid, propionic acid, butyric acid, 2-ethylhexanoic acid, trimethylhexanoic acid, 2-propylheptanoic acid, isononanoic acid, versatic acids, decanoic acid, undecanoic acid, dodecanoic acid, saturated or unsaturated fatty acids with 12 to 24 carbon atoms, or mixtures thereof, salicylic acid, oxalic acid mono-C₁-C₄-alkyl ester, phthalic acid mono-C₁-C₄-alkyl ester, C₁₂-C₁₀₀-alkyl- and -alkenyl succinic acid, especially dodecenyl succinic acid, hexadecenyl succinic acid, eicosenyl succinic acid, and polyisobutenyl succinic acid. Further examples are methyl carbonate, ethyl carbonate, n-butyl carbonate, 2-hydroxyethyl carbonate, and 2-hydroxypropyl carbonate.

In one preferred embodiment the nitrogen compounds quaternized in the presence of an acid or in an acid-free manner are obtainable by addition of a compound which comprises at least one oxygen- or nitrogen-containing group reactive with an anhydride and additionally at least one quaternizable amino group onto a polycarboxylic anhydride compound and subsequent quaternization, especially with an epoxide, e.g. styrene or propylene oxide, in the absence of free acid, as described in WO 2012/004300, or with a carboxylic ester, e.g. dimethyl oxalate or methyl salicylate. Suitable compounds having at least one oxygen- or nitrogen-containing group reactive with anhydride and additionally at least one quaternizable amino group are especially polyamines having at least one primary or secondary amino group and at least one tertiary amino group, especially N,N-dimethyl-1 ,3-propane diamine, N,N-dimethyl-1 ,2-ethane diamine or N,N, N'-trimethyl-1,2-ethane diamine. Useful polycarboxylic anhydrides are especially dicarboxylic acids such as succinic acid, having a relatively long-chain hydrocarbyl substituent, preferably having a number-average molecular weight Mₙ for the hydrocarbyl substituent of 200 to 10.000, in particular of 350 to 5000. Such a quaternized nitrogen compound is, for example, the reaction product, obtained at 40°C, of polyisobutenylsuccinic anhydride, in which the polyisobutenyl radical typically has an Mₙ of 1000, with 3-(dimethylamino)propylamine, which constitutes a polyisobutenylsuccinic monoamide and which is subsequently quaternized with dimethyl oxalate or methyl salicylate or with styrene oxide or propylene oxide in the absence of free acid.

Further quaternized nitrogen compounds suitable as compounds are described in
WO 2006/135881 A1, page 5, line 13 to page 12, line 14;
WO 10/132259 A1, page 3, line 28 to page 10, line 25;
WO 2008/060888 A2, page 6, line 15 to page 14, line 29;
WO 2011/095819 A1, page 4, line 5 to page 9, line 29;
GB 2496514 A, paragraph [00012] to paragraph [00041];
WO 2013/117616 A1, page 3, line 34 to page 11, line 2;
WO 14/202425 A2, page 3, line 14 to page 5, line 9;
WO 14/195464 A1, page 15, line 31 to page 45, line 26 and page 75, lines 1 to 4;
WO 15/040147 A1, page 4, line 34 to page 5, line 18 and page 19, line 11 to page 50, line 10;
WO 14/064151 A1, page 5, line 14 to page 6, line 17 and page 16, line 10 to page 18, line 12;
WO 2013/064689 A1, page 18, line 16 to page 29, line 8; and
WO 2013/087701 A1, page 13, line 25 to page 19, line 30,
WO 13/000997 A1, page 17, line 4 to page 25, line 3,
WO 12/004300, page 5, lines 20 to 30, page 8, line 1 to page 10, line 10, and page 19, line 29 to page 28, line 3.

In one embodiment the quaternized ammonium compound is of formula 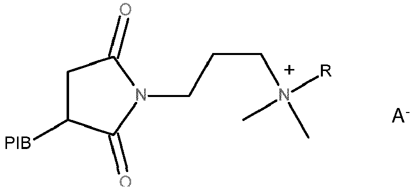 wherein in this formula
PIB stands for a polyisobutenyl residue having a number average molecular weight Mₙ of from 550 to 2300, preferably from 650 to 1500 and more preferably from 750 to 1300 g/mol,
R stands for an C₁- to C₄-alkyl or hydroxy-C₁- to C₄-alkyl, preferably methyl or 2-hydroxypropyl, and
A⁻ stands for an anion, preferably carboxylate R⁸COO⁻ or a carbonate R⁸O-COO⁻ as defined above, more preferably acetate, salicylate or methyloxalate.

In another preferred embodiment the quaternized ammonium compound is of formula 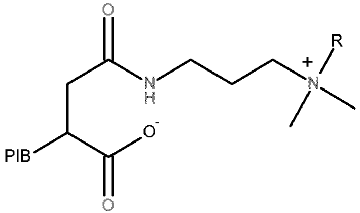 wherein in this formula
PIB stands for a polyisobutenyl residue having a number average molecular weight Mₙ of from 550 to 2300, preferably from 650 to 1500 and more preferably from 750 to 1300 g/mol,
R stands for a hydroxy-C₁- to C₄-alkyl, preferably 2-hydroxypropyl.

In another embodiment the quaternized compound is of formula 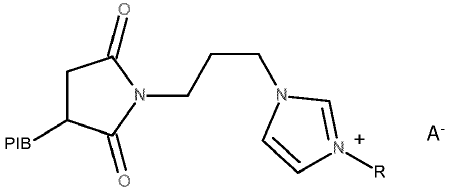 wherein in this formula
PIB stands for a polyisobutenyl residue having a number average molecular weight Mₙ of from 550 to 2300, preferably from 650 to 1500 and more preferably from 750 to 1300 g/mol,
R stands for an C₁- to C₄-alkyl or hydroxy-C₁- to C₄-alkyl, preferably methyl, and
A stands for an anion, preferably carboxylate R⁸COO⁻ or a carbonate R⁸O-COO⁻ as defined above, more preferably salicylate or methyloxalate.

In another embodiment the quaternized ammonium compound is of formula 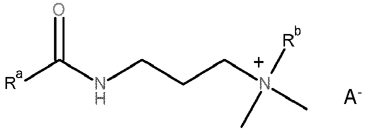 wherein in this formula
R^{a} stands for C₁-C₂₀-alkyl, preferably C₉- to C₁₇-alkyl, more preferably for undecyl, tridecyl, pentadecyl or heptadecyl,
R^{b} stands for a hydroxy-C₁- to C₄-alkyl, preferably 2-hydroxypropyl or 2-hydroxybutyl, and
A stands for an anion, preferably carboxylate R⁸COO⁻, as defined above, more preferably R⁸COO⁻ being a carboxylate of a fatty acid, especially A being acetate, 2-ethylhexanoate, oleate, polyisobutenyl succinate or monoesters of polyisobutenyl succinate.

In one embodiment the quaternized ammonium compound is of formula 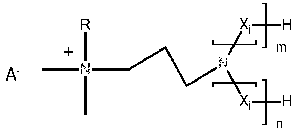 wherein in this formula
Xᵢ for i ₌ 1 to n and 1 to m are independently of another selected from the group consisting of - CH₂-CH₂-O-, -CH₂-CH(CH₃)-O-, -CH(CH₃)-CH₂-O-, -CH₂-C(CH₃)₂-O-, -C(CH₃)₂-CH₂-O-, -CH₂-CH(C₂H₅)-O-, -CH(C₂H₅)-CH₂-O- and -CH(CH₃)-CH(CH₃)-O-, preferably selected from the group consisting of -CH₂-CH(CH₃)-O-, -CH(CH₃)-CH₂-O-, -CH₂-C(CH₃)₂-O-, -C(CH₃)₂-CH₂-O-, -CH₂-CH(C₂H₅)-O-, -CH(C₂H₅)-CH₂-O- and -CH(CH₃)-CH(CH₃)-O-, more preferably selected from the group consisting of -CH₂-CH(CH₃)-O-, -CH(CH₃)-CH₂-O-, -CH₂-C(CH₃)₂-O-, -C(CH₃)₂-CH₂-O-, - CH₂-CH(C₂H₅)-O- and -CH(C₂H₅)-CH₂-O-, most preferably selected from the group consisting of -CH₂-CH(C₂H₅)-O-, -CH(C₂H₅)-CH₂-O-, -CH₂-CH(CH₃)-O- and -CH(CH₃)-CH₂-O-, and especially selected from the group consisting of -CH₂-CH(CH₃)-O- and -CH(CH₃)-CH₂-O-,
m and n independently of another are positive integers, with the proviso that the sum (m + n) is from 2 to 50, preferably from 5 to 40, more preferably from 10 to 30, and especially from 15 to 25,
R stands for an C₁- to C₄-alkyl, preferably methyl, and
A stands for an anion, preferably carboxylate R⁸COO⁻ or a carbonate R⁸O-COO⁻ as defined above, more preferably salicylate or methyloxalate.

In another preferred embodiment the quaternized ammonium compound is of formula 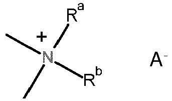 wherein in this formula
R^{a} and R^{b} independently of another stand for C₁-C₂₀-alkyl or hydroxy-C₁- to C₄-alkyl, preferably R^{a} stands for C₁-C₂₀-alkyl, preferably ethyl, n-butyl, n-octyl, n-dodecyl, tetradecyl or hexadecyl, and R^{b} stands for hydroxy-C₁- to C₄-alkyl, preferably 2-hydroxypropyl,
A stands for an anion, preferably carboxylate R⁸COO⁻ or a carbonate R⁸O-COO⁻ as defined above, more preferably C₁₂-C₁₀₀-alkyl- and -alkenyl succinic acid, especially dodecenyl succinic acid, hexadecenyl succinic acid, eicosenyl succinic acid, and polyisobutenyl succinic acid.

### Mannich adducts

Typical Mannich adducts are described in US 8449630 B2, preferred are Mannich adducts according to formula I of US 8449630 B2.

In a preferred embodiment the Mannich adducts are obtainable as described in US 8449630 B2, column 7, line 35 to column 9, line 52.

Preferably the Mannich adducts are obtainable by reaction of
- at least one hydrocarbyl-substituted phenol, preferably a phenol of formula V of US 8449630 B2, more preferably para hydrocarbyl-substituted phenol or para hydrocarbyl-substituted ortho-cresol, with
- at least one aldehyde, preferably acetaldehyde or formaldehyde, more preferably formaldehyde, and
- at least one amine according to variant 2 of US 8449630 B2, preferably selected from the group consisting of octylamine, 2-ethylhexylamine, nonylamine, decylamine, undecylamine, dodecylamine, tridecylamine, tetradecylamine, pentadecylamine, hexadecylamine, heptadecylamine, octadecylamine, nonadecylamine, eicosylamine, cyclooctylamine, cyclodecylamine di-n-butylamine, diisobutylamine, di-tert-butylamine, dipentylamine, dihexylamine, diheptylamine, dioctylamine, di(2-ethylhexylamine), dinonylamine, didecylamine, N-methylcyclohexylamine, N-ethylcyclohexylamine, dicyclohexylamine, triethylenetetramine, tetraethylenepentamine, pentaethylenehexamine, dipropylenetriamine, tripropylenetetramine, tetrapropylenepentamine, dibutylenetriamine, tributylenetetramine, tetrabutylenepentamine, N, N-dipropylmethylenediamine, N, N-dipropylethylene-1, 2-diamine, N, N-dimethylpropylene- 1, 3-diamine, N, N-diethylpropylene- 1, 3-diamine, N, N-dipropylpropylene-1, 3-diamine, N, N-diethylbutylene-1, 4-diamine, N, N-dipropylbutylene-1, 4-diamine, N, N-dimethylpentylene-1, 3-diamine, N, N-diethylpentylene- 1, 5-diamine, N, N-dipropylpentylene-1, 5-diamine, N, N-dimethylhexylene-1, 6-diamine, N, N-diethylhexylene- 1, 6-diamine, N, N-dipropylhexylene-1, 6-diamine, bis [2-(N,N-dimethylamino)ethyl] amine, bis [2-(N,N-dipropylamino)ethyl]amine, bis[3-(N, N-dimethylamino)propyl]amine, bis[3-(N, N-diethylamino)-propyl]amine, bis [3-(N,N-dipropylamino)propyl] amine, bis[4-(N, Ndimethylamino) butyl]amine, bis[4-(N, N-diethylamino) butyl]amine, bis[4-(N, N-dipropylamino)butyl]amine, bis[5-(N, N-dimethylamino)-pentyl] amine, bis[5-(N, N-diethylamino)pentyl]amine, bis[5-(N, N-dipropylamino)pentyl]amine, bis[6-(N, N-dimethylamino)-hexyl]amine, bis [6-(N,N-diethylamino)hexyl] amine, bis[6-(N, N-dipropylamino) hexyl]amine, tris[2-(N, N-dimethylamino) ethyl]amine, tris[2-(N, N-dipropylamino)ethyl]amine, tris[3-(N, N-dimethylamino)propyl] amine, tri s [3-(N,Ndiethylamino)propyl]amine, tris[3-(N, N-dipropylamino)propyl]amine, tris[4-(N, N-dimethylamino)butyl]amine, tris[4-(N,N-diethylamino)-butyl] amine, tris[4-(N, Ndipropylamino)butyl]amine, tris[5-(N, N-dimethylamino)pentyl]amine, tris[5-(N, N-diethylamino)pentyl]amine, tris[5-(N,N-dipropylamino)pentyl]amine, tris[6-(N, N-dimethylamino)hexyl]amine, tris[6-(N, N-diethylamino)-hexyl]amine, and tris[6-(N, N-dipropylamino)hexyl]amine,
more preferably selected from the group consisting of dimethylamine, diethylamine, di-n-butylamine, diethylenetriamine, triethylenetetramine, tetraethylenepentamine, pentaethylenehexamine, N, N-dimethylpropylene- 1, 3-diamine, and N, N-diethylpropylene- 1, 3-diamine.

The hydrocarbyl residue of the at least one hydrocarbyl-substituted phenol preferably has a number average molecular weight Mn of from 85 to 5000, preferably of from 113 to 2500, more preferably of from 550 to 1500, and especially from 750 to 1100.

In a preferred embodiment the hydrocarbyl residue is a polyisobutene radical of the before-mentioned molecular weight, more preferably derived from a "reactive" polyisobutene radical as defined in US 8449630 B2.

In a preferred embodiment the Mannich adduct is of formula 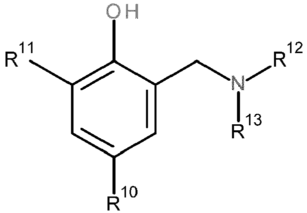 or of formula 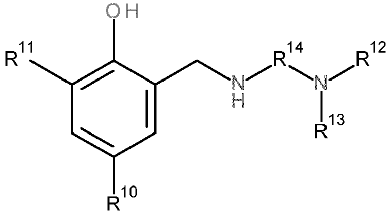 wherein
R¹⁰ is a hydrocarbyl residue with a number average molecular weight Mn of from 85 to 5000, preferably of from 113 to 2500, more preferably of from 550 to 1500, and most preferably of from 750 to 1100, and especially is a polyisobutene radical of the before-mentioned molecular weight, more preferably derived from a "reactive" polyisobutene radical,
R¹¹ is hydrogen, methyl, ethyl, iso-propyl, n-butyl, tert-butyl, but-2-yl, or amyl, preferably hydrogen or methyl, and more preferably methyl,
R¹² and R¹³ independently of another are C₁- to C₆-alkyl, preferably C₁- to C₄-alkyl, more preferably are methyl, ethyl, n-propyl, iso-propyl, n-butyl, tert-butyl, even more preferably are methyl, ethyl or n-butyl, or R¹² and R¹³ together the nitrogen atom form a five- or six-membered ring, preferably a pyrrolidine, piperidine or morpholine ring, and
R¹⁴ is bivalent alkylene residue having 1 to 6 carbon atoms, preferably 1 to 4 carbon atoms, more preferably 2 or 3 carbon atoms, most preferably selected from the group consisting of methylene, 1,2-ethylene, 1,2-propylene, 1,3-propylene, 1,4-butylene, and 1,6-hexylene, and especially being 1,2-ethylene or 1,3-propylene.

### Polvalkenemono- or polyalkenepolyamines

Polyalkenemono- or polyalkenepolyamines are preferably based on polypropene or on high-reactivity (i.e. having predominantly terminal double bonds) or conventional (i.e. having predominantly internal double bonds) polybutene or especially polyisobutene with M_{n =} 300 to 5000, more preferably 500 to 2500 and especially 700 to 2500. Such additives based on high-reactivity polyisobutene, which can be prepared from the polyisobutene which may comprise up to 20% by weight of n-butene units by hydroformylation and reductive amination with ammonia, monoamines or polyamines such as dimethylaminopropylamine, ethylenediamine, diethylenetriamine, triethylenetetramine or tetraethylenepentamine, are known especially from EP-A 244 616. When polybutene or polyisobutene having predominantly internal double bonds (usually in the β and γ positions) are used as starting materials in the preparation of the additives, a possible preparative route is by chlorination and subsequent amination or by oxidation of the double bond with air or ozone to give the carbonyl or carboxyl compound and subsequent amination under reductive (hydrogenating) conditions. The amines used here for the amination may be, for example, ammonia, monoamines or the abovementioned polyamines. Corresponding additives based on polypropene are described more particularly in WO-A 94/24231.

Further particular additives comprising monoamino groups are the hydrogenation products of the reaction products of polyisobutenes having an average degree of polymerization P = 5 to 100 with nitrogen oxides or mixtures of nitrogen oxides and oxygen, as described more particularly in WO-A 97/03946.

Further particular additives comprising monoamino groups are the compounds obtainable from polyisobutene epoxides by reaction with amines and subsequent dehydration and reduction of the amino alcohols, as described more particularly in DE-A 196 20 262.

Examples of particularly useful polyalkylene radicals are polyisobutenyl radicals derived from what are called "high-reactivity" polyisobutenes which feature a high content of terminal double bonds. Terminal double bonds are alpha-olefinic double bonds of the type 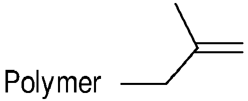 which are also referred to collectively as vinylidene double bonds. Suitable high-reactivity polyisobutenes are, for example, polyisobutenes which have a proportion of vinylidene double bonds of greater than 70 mol%, especially greater than 80 mol% or greater than 85 mol%. Preference is given especially to polyisobutenes which have homogeneous polymer skeletons. Homogeneous polymer skeletons are possessed especially by those polyisobutenes formed from isobutene units to an extent of at least 85% by weight, preferably to an extent of at least 90% by weight and more preferably to an extent of at least 95% by weight. Such high-reactivity polyisobutenes preferably have a number-average molecular weight within the abovementioned range. In addition, the high-reactivity polyisobutenes may have a polydispersity in the range from 1.05 to 7, especially of about 1.1 to 2.5, for example of less than 1.9 or less than 1.5. Polydispersity is understood to mean the quotient of weight-average molecular weight Mw divided by the number-average molecular weight Mn.

Particularly suitable high-reactivity polyisobutenes are, for example, the Glissopal brands from BASF SE, especially Glissopal^{®} 1000 (Mn = 1000), Glissopal^{®} V 33 (Mn = 550) and Glissopal^{®} 2300 (Mn = 2300), and mixtures thereof. Other number-average molecular weights can be established in a manner known in principle by mixing polyisobutenes of different number-average molecular weights or by extractive enrichment of polyisobutenes of particular molecular weight ranges.

Due to their high proportion of vinylidene double bonds these polyisobutenes are especially reactive to undergo hydroformylation and subsequent amination, preferably with ammonia, to yield the corresponding polyisobutene amines, which represent a preferred embodiment of the present invention.

### Corrosion inhibitors

As corrosion inhibitors in principle all compounds known in the art for application in fuels may be used.

Suitable corrosion inhibitors are, for example, succinic esters or hemiesters, in particular with polyols, fatty acid derivatives, for example oleic esters, oligomerized fatty acids, such as dimeric fatty acid, substituted ethanolamines, and products sold under the trade name RC 4801 (Rhein Chemie Mannheim, Germany) or HiTEC 536 (Afton Corporation).

According to US 6043199 the latter is believed to be a reaction product of linear or branched alkyl or alkenyl substituted succinic anhydride with substituted amino-imidazolines resulting in what are believed to be linear or branched alkyl or alkenyl substituted succinimide or amine substituted succinimides.

In a preferred embodiment the corrosion inhibitor is selected from the group consisting of
- fatty acids or fatty acid derivatives, preferably oleic acid or its esters,
- oligomerized fatty acids, preferably dimeric fatty acid, more preferably dimeric oleic acid (CAS: 61788-89-4),
- alkyl or alkenyl substituted succinic acids, esters or hemiesters, and
- olefin-carboxylic acid copolymers (see below).

In a more preferred embodiment the corrosion inhibitor is selected from the group consisting of
- oligomerized fatty acids, preferably dimeric fatty acid, more preferably dimeric oleic acid (CAS: 61788-89-4),
- alkyl or alkenyl substituted succinic acids, esters or hemiesters, and
- olefin-carboxylic acid copolymers (see below).

### Alkyl or alkenyl substituted succinic acids, esters or hemiesters

The succinic acids, esters or hemiesters are preferably substituted with C₈- to C₁₀₀-alkyl or
- alkenyl radicals.

In a preferred embodiment the succinic acids or hemiesters follow formula 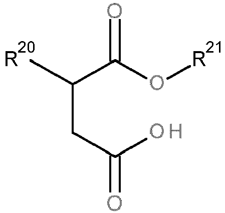 wherein
R²⁰ is a C₈- to C₁₀₀-alkyl or C₈- to C₁₀₀-alkenyl group, preferably C₈- to C₁₀₀-alkenyl, more preferably C₁₂- to C₉₀-alkenyl, and even more preferably C₁₆- to C₈₀-alkenyl group, and
R²¹ is hydrogen or C₁- to C₂₀-alkyl or C₂- to C₄-hydroxyalkyl, preferably hydrogen.

The underlying succinic acid anhydrides are obtainable by thermal ene reaction of C₈- to C₁₀₀-alkenes, preferably oligomers or polymers of propene, 1-butene or isobutene, with maleic anhydride. The above-mentioned corrosion inhibitors are obtainable from such anhydrides by hydrolysis or reaction with the appropriate alcohol.

### Olefin-carboxylic acid copolymers

The olefin-carboxylic acid copolymer (A) is a copolymer obtainable by
- in a first reaction step (I) copolymerizing
   (Aa) at least one ethylenically unsaturated mono- or dicarboxylic acid or derivatives thereof, preferably a dicarboxylic acid,
   (Ab) at least one α-olefin having from at least 12 up to and including 30 carbon atoms,
   (Ac) optionally at least one further aliphatic or cycloaliphatic olefin which has at least 4 carbon atoms and is different than (Ab) and
   (Ad) optionally one or more further copolymerizable monomers other than monomers (Aa), (Ab) and (Ac), selected from the group consisting of
   (Ada) vinyl esters,
   (Adb) vinyl ethers,
   (Adc) (meth)acrylic esters of alcohols having at least 5 carbon atoms,
   (Add) allyl alcohols or ethers thereof,
   (Ade) N-vinyl compounds selected from the group consisting of vinyl compounds of heterocycles containing at least one nitrogen atom, N-vinylamides or N-vinyllactams,
   (Adf) ethylenically unsaturated aromatics,
   (Adg) α,β-ethylenically unsaturated nitriles,
   (Adh) (meth)acrylamides and
   (Adi) allylamines,
   followed by
- in a second optional reaction step (II) partly or fully hydrolyzing and/or saponifying anhydride or carboxylic ester functionalities present in the copolymer obtained from (I), the second reaction step being run at least when the copolymer obtained from reaction step (I) does not comprise any free carboxylic functionalities.

### Description of the copolymer (A)

The monomer (Aa) is at least one, preferably one to three, more preferably one or two and most preferably exactly one ethylenically unsaturated, preferably α,β-ethylenically unsaturated, mono- or dicarboxylic acid(s) or derivatives thereof, preferably a dicarboxylic acid or derivatives thereof.

Derivatives are understood to mean
- the corresponding anhydrides in monomeric or else polymeric form,
- mono- or dialkyl esters, preferably mono- or di-C₁-C₄-alkyl esters, more preferably mono- or dimethyl esters or the corresponding mono- or diethyl esters, and
- mixed esters, preferably mixed esters having different C₁-C₄ alkyl components, more preferably mixed methyl ethyl esters.

Preferably, the derivatives are anhydrides in monomeric form or di-C₁-C₄-alkyl esters, more preferably anhydrides in monomeric form.

In the context of this document, C₁-C₄-alkyl is understood to mean methyl, ethyl, iso-propyl, n-propyl, n-butyl, iso-butyl, sec-butyl and tert-butyl, preferably methyl and ethyl, more preferably methyl.

Examples of α,β-ethylenically unsaturated mono- or dicarboxylic acids are those mono- or dicarboxylic acids or derivatives thereof in which the carboxyl group or, in the case of dicarboxylic acids, at least one carboxyl group, preferably both carboxyl groups, is/are conjugated to the ethylenically unsaturated double bond.

Examples of ethylenically unsaturated mono- or dicarboxylic acids that are not α,β-ethylenically unsaturated are cis-5-norbornene-endo-2,3-dicarboxylic anhydride, exo-3,6-epoxy-1,2,3,6-tetrahydrophthalic anhydride and cis-4-cyclohexene-1,2-dicarboxylic anhydride.

Examples of a,β-ethylenically unsaturated monocarboxylic acids are acrylic acid, methacrylic acid, crotonic acid and ethylacrylic acid, preferably acrylic acid and methacrylic acid, referred to in this document as (meth)acrylic acid for short, and more preferably acrylic acid.

Particularly preferred derivatives of a,β-ethylenically unsaturated monocarboxylic acids are methyl acrylate, ethyl acrylate, n-butyl acrylate and methyl methacrylate.

Examples of dicarboxylic acids are maleic acid, fumaric acid, itaconic acid (2-methylenebutanedioic acid), citraconic acid (2-methylmaleic acid), glutaconic acid (pent-2-ene-1,5-dicarboxylic acid), 2,3-dimethylmaleic acid, 2-methylfumaric acid, 2,3-dimethylfumaric acid, methylenemalonic acid and tetrahydrophthalic acid, preferably maleic acid and fumaric acid and more preferably maleic acid and derivatives thereof.

More particularly, monomer (Aa) is maleic anhydride.

Monomer (Ab) is at least one, preferably one to four, more preferably one to three, even more preferably one or two and most preferably exactly one α-olefin(s) having from at least 12 up to and including 30 carbon atoms. The α-olefins (Ab) preferably have at least 14, more preferably at least 16 and most preferably at least 18 carbon atoms. Preferably, the α-olefins (Ab) have up to and including 28, more preferably up to and including 26 and most preferably up to and including 24 carbon atoms.

Preferably, the α-olefins may be one or more linear or branched, preferably linear, 1-alkene.

Examples of these are 1-dodecene, 1-tridecene, 1-tetradecene, 1-pentadecene, 1-hexadecene, 1-heptadecene, 1- octadecene, 1-nonodecene, 1-eicosene, 1-docosene, 1-tetracosene, 1-hexacosene, preference being given to 1-octadecene, 1-eicosene, 1-docosene and 1-tetracosene, and mixtures thereof.

Further examples of α-olefin (Ab) are those olefins which are oligomers or polymers of C₂ to C₁₂ olefins, preferably of C₃ to C₁₀ olefins, more preferably of C₄ to C₆ olefins. Examples thereof are ethene, propene, 1-butene, 2-butene, isobutene, pentene isomers and hexene isomers, preference being given to ethene, propene, 1-butene, 2-butene and isobutene.

Named examples of α-olefins (Ab) include oligomers and polymers of propene, 1-butene, 2-butene, isobutene, and mixtures thereof, particularly oligomers and polymers of propene or iso-butene or of mixtures of 1-butene and 2-butene. Among the oligomers, preference is given to the trimers, tetramers, pentamers and hexamers, and mixtures thereof.

In addition to the olefin (Ab), it is optionally possible to incorporate at least one, preferably one to four, more preferably one to three, even more preferably one or two and especially exactly one further aliphatic or cycloaliphatic olefin(s) (Ac) which has/have at least 4 carbon atoms and is/are different than (Ab) by polymerization into the inventive copolymer.

The olefins (Ac) may be olefins having a terminal (α-)double bond or those having a non-terminal double bond, preferably having an α-double bond. The olefin (Ac) preferably comprises olefins having 4 to fewer than 12 or more than 30 carbon atoms. If the olefin (Ac) is an olefin having 12 to 30 carbon atoms, this olefin (Ac) does not have an α-double bond.

Examples of aliphatic olefins (Ac) are 1-butene, 2-butene, isobutene, pentene isomers, hexene isomers, heptene isomers, octene isomers, nonene isomers, decene isomers, undecene isomers and mixtures thereof.

Examples of cycloaliphatic olefins (Ac) are cyclopentene, cyclohexene, cyclooctene, cyclode-cene, cyclododecene, α- or β-pinene and mixtures thereof, limonene and norbornene.

Further examples of olefins (Ac) are polymers having more than 30 carbon atoms of propene, 1-butene, 2-butene or isobutene or of olefin mixtures comprising the latter, preferably of isobutene or of olefin mixtures comprising the latter, more preferably having a mean molecular weight M_{w} in the range from 500 to 5000 g/mol, preferably 650 to 3000 and more preferably 800 to 1500 g/mol.

Preferably, the oligomers or polymers comprising isobutene in copolymerized form have a high content of terminal ethylenic double bonds (a-double bonds), for example at least 50 mol%, preferably at least 60 mol%, more preferably at least 70 mol% and most preferably at least 80 mol%.

For the preparation of such oligomers or polymers comprising isobutene in copolymerized form, suitable isobutene sources are either pure isobutene or isobutene-containing C4 hydrocarbon streams, for example C4 raffinates, especially "raffinate 1", C4 cuts from isobutane dehydrogenation, C4 cuts from steamcrackers and from FCC crackers (fluid catalyzed cracking), provided that they have substantially been freed of 1,3-butadiene present therein. A C4 hydrocarbon stream from an FCC refinery unit is also known as a "b/b" stream. Further suitable isobutene-containing C4 hydrocarbon streams are, for example, the product stream of a propylene-isobutane cooxidation or the product stream from a metathesis unit, which are generally used after customary purification and/or concentration. Suitable C4 hydrocarbon streams comprise generally less than 500 ppm, preferably less than 200 ppm, of butadiene. The presence of 1-butene and of cis- and trans-2-butene is substantially uncritical. Typically, the isobutene concentration in said C4 hydrocarbon streams is in the range from 40% to 60% by weight. For instance, raffinate 1 generally consists essentially of 30% to 50% by weight of isobutene, 10% to 50% by weight of 1-butene, 10% to 40% by weight of cis- and trans-2-butene and 2% to 35% by weight of butanes; in the polymerization process the unbranched butenes in the raffinate 1 are generally virtually inert, and only the isobutene is polymerized.

In a preferred embodiment, the monomer source used for polymerization is a technical C4 hydrocarbon stream having an isobutene content of 1% to 100% by weight, especially of 1% to 99% by weight, in particular of 1% to 90% by weight, more preferably of 30% to 60% by weight, especially a raffinate 1 stream, a b/b stream from an FCC refinery unit, a product stream from a propylene-isobutane cooxidation or a product stream from a metathesis unit.

Especially when a raffinate 1 stream is used as isobutene source, the use of water as the sole initiator or as further initiator has been found to be useful, particularly when polymerization is effected at temperatures of -20°C to +30°C, especially of 0°C to +20°C. At temperatures of - 20°C to +30°C, especially of 0°C to +20°C, however, it is possible to dispense with the use of an initiator when using a raffinate 1 stream as isobutene source.

Said isobutene-containing monomer mixture may comprise small amounts of contaminants such as water, carboxylic acids or mineral acids without causing any critical yield or selectivity losses. It is appropriate to the purpose to avoid accumulation of these impurities by removing such harmful substances from the isobutene-containing monomer mixture, for example, by adsorption on solid adsorbents such as activated carbon, molecular sieves or ion exchangers.

It is also possible, albeit less preferable, to convert monomer mixtures of isobutene or of the isobutene-containing hydrocarbon mixture with olefinically unsaturated monomers copolymerizable with isobutene. If monomer mixtures of isobutene with suitable comonomers are to be copolymerized, the monomer mixture comprises preferably at least 5% by weight, more preferably at least 10% by weight and especially at least 20% by weight of isobutene, and preferably at most 95% by weight, more preferably at most 90% by weight and especially at most 80% by weight of comonomers.

In a preferred embodiment, the mixture of the olefins (Ab) and optionally (Ac), averaged to their molar amounts, have at least 12 carbon atoms, preferably at least 14, more preferably at least 16 and most preferably at least 17 carbon atoms.

For example, a 2:3 mixture of docosene and tetradecene has an averaged value for the carbon atoms of 0.4 _{×} 22 + 0.6 _{×} 14 ₌ 17.2.

The upper limit is less relevant and is generally not more than 60 carbon atoms, preferably not more than 55, more preferably not more than 50, even more preferably not more than 45 and especially not more than 40 carbon atoms.

The optional monomer (Ad) is at least one monomer, preferably one to three, more preferably one or two and most preferably exactly one monomer(s) selected from the group consisting of
(Ada) vinyl esters,
(Adb) vinyl ethers,
(Adc) (meth)acrylic esters of alcohols having at least 5 carbon atoms,
(Add) allyl alcohols or ethers thereof,
(Ade) N-vinyl compounds selected from the group consisting of vinyl compounds of heterocycles containing at least one nitrogen atom, N-vinylamides or N-vinyllactams,
(Adf) ethylenically unsaturated aromatics and
(Adg) α,β-ethylenically unsaturated nitriles,
(Adh) (meth)acrylamides and
(Adi) allylamines.

Examples of vinyl esters (Ada) are vinyl esters of C₂- to C₁₂-carboxylic acids, preferably vinyl acetate, vinyl propionate, vinyl butyrate, vinyl pentanoate, vinyl hexanoate, vinyl octanoate, vinyl 2-ethylhexanoate, vinyl decanoate, and vinyl esters of Versatic Acids 5 to 10, preferably vinyl esters of 2,2-dimethylpropionic acid (pivalic acid, Versatic Acid 5), 2,2-dimethylbutyric acid (neohexanoic acid, Versatic Acid 6), 2,2-dimethylpentanoic acid (neoheptanoic acid, Versatic Acid 7), 2,2-dimethylhexanoic acid (neooctanoic acid, Versatic Acid 8), 2,2-dimethylheptanoic acid (neononanoic acid, Versatic Acid 9) or 2,2-dimethyloctanoic acid (neodecanoic acid, Versatic Acid 10).

Examples of vinyl ethers (Adb) are vinyl ethers of C₁- to C₁₂-alkanols, preferably vinyl ethers of methanol, ethanol, iso-propanol, n-propanol, n-butanol, iso-butanol, sec-butanol, tert-butanol, n-hexanol, n-heptanol, n-octanol, n-decanol, n-dodecanol (lauryl alcohol) or 2-ethylhexanol.

Preferred (meth)acrylic esters (Adc) are (meth)acrylic esters of C₅- to C₁₂-alkanols, preferably of n-pentanol, n-hexanol, n-heptanol, n-octanol, n-decanol, n-dodecanol (lauryl alcohol), 2-ethylhexanol or 2-propylheptanol. Particular preference is given to pentyl acrylate, 2-ethylhexyl acrylate, 2-propylheptyl acrylate.

Examples of monomers (Add) are allyl alcohols and allyl ethers of C₂- to C₁₂-alkanols, preferably allyl ethers of methanol, ethanol, *iso*-propanol, n-propanol, n-butanol, *iso*-butanol, sec-butanol, *tert*-butanol, n-hexanol, n-heptanol, n-octanol, n-decanol, n-dodecanol (lauryl alcohol) or 2-ethylhexanol.

Examples of vinyl compounds (Ade) of heterocycles comprising at least one nitrogen atom are N-vinylpyridine, N-vinylimidazole and N-vinylmorpholine.

Preferred compounds (Ade) are N-vinylamides or N-vinyllactams.

Examples of N-vinylamides or N-vinyllactams (Ade) are N-vinylformamide, N-vinylacetamide, N-vinylpyrrolidone and N-vinylcaprolactam.

Examples of ethylenically unsaturated aromatics (Adf) are styrene and α-methylstyrene.

Examples of a,β-ethylenically unsaturated nitriles (Adg) are acrylonitrile and methacrylonitrile.

Examples of (meth)acrylamides (Adh) are acrylamide and methacrylamide.

Examples of allylamines (Adi) are allylamine, dialkylallylamine and trialkylallylammonium halides.

Preferred monomers (Ad) are (Ada), (Adb), (Adc), (Ade) and/or (Adf), more preferably (Ada), (Adb) and/or (Adc), even more preferably (Ada) and/or (Adc) and especially (Adc).

The incorporation ratio of the monomers (Aa) and (Ab) and optionally (Ac) and optionally (Ad) in the polymer obtained from reaction step (I) is generally as follows:
The molar ratio of (Aa)/((Ab) and (Ac)) (in total) is generally from 10:1 to 1:10, preferably 8:1 to 1:8, more preferably 5:1 to 1:5, even more preferably 3:1 to 1:3, particularly 2:1 to 1:2 and especially 1.5:1 to 1:1.5. In the preferred particular case of maleic anhydride as monomer (Aa), the molar incorporation ratio of maleic anhydride to monomers ((Ab) and (Ac)) (in total) is about 1:1.

The molar ratio of obligatory monomer (Ab) to monomer (Ac), if present, is generally of 1:0.05 to 10, preferably of 1:0.1 to 6, more preferably of 1:0.2 to 4, even more preferably of 1:0.3 to 2.5 and especially 1:0.5 to 1.5.

In a preferred embodiment, no optional monomer (Ac) is present in addition to monomer (Ab).

The proportion of one or more of the monomers (Ad), if present, based on the amount of the monomers (Aa), (Ab) and optionally (Ac) (in total) is generally 5 to 200 mol%, preferably 10 to 150 mol%, more preferably 15 to 100 mol%, even more preferably 20 to 50 mol% and especially 0 to 25 mol%.

In a preferred embodiment, no optional monomer (Ad) is present.

In a second reaction step (II), the anhydride or carboxylic ester functionalities present in the copolymer obtained from (I) are partly or fully hydrolyzed and/or saponified.

Reaction step (II) is obligatory in case the copolymer obtained from reaction step (I) does not comprise free carboxylic acid groups.

Hydrolization of anhydride groups is preferred over saponification of ester groups.

Preferably, 10% to 100% of the anhydride or carboxylic ester functionalities present are hydrolyzed and/or saponified, preferably at least 20%, more preferably at least 30%, even more preferably at least 50% and particularly at least 75% and especially at least 85%.

For a hydrolysis, based on the anhydride functionalities present, the amount of water that corresponds to the desired hydrolysis level is added and the copolymer obtained from (I) is heated in the presence of the added water. In general, a temperature of preferably 20 to 150°C is sufficient for the purpose, preferably 60 to 100°C. If required, the reaction can be conducted under pressure in order to prevent the escape of water. Under these reaction conditions, in general, the anhydride functionalities in the copolymer are converted selectively, whereas any carboxylic ester functionalities present in the copolymer react at least only to a minor degree, if at all.

For a saponification, the copolymer is reacted with an amount of a strong base corresponding to the desired saponification level in the presence of water.

Strong bases used may preferably be hydroxides, oxides, carbonates or hydrogencarbonates of alkali metals or alkaline earth metals.

The copolymer obtained from (I) is then heated in the presence of the added water and the strong base. In general, a temperature of preferably 20 to 130°C is sufficient for the purpose, preferably 50 to 110°C. If required, the reaction can be conducted under pressure.

It is also possible to hydrolyze the carboxylic ester functionalities with water in the presence of an acid. Acids used are preferably mineral acids, carboxylic acids, sulfonic acids or phosphorus acids having a pKa of not more than 5, more preferably not more than 4.

Examples are acetic acid, formic acid, oxalic acid, salicylic acid, substituted succinic acids, aromatically substituted or unsubstituted benzenesulfonic acids, sulfuric acid, nitric acid, hydrochloric acid or phosphoric acid; the use of acidic ion exchange resins is also conceivable.

In a preferred embodiment for anhydrides, especially maleic anhydride being monomers (Aa), such anhydride moieties are partly or fully, especially fully hydrolysed while potentially existing ester groups in the copolymer remain intact. In this case no saponification in step (II) takes place.

The copolymer obtained from (I) is then heated in the presence of the added water and the acid. In general, a temperature of preferably 40 to 200°C is sufficient for the purpose, preferably 80 to 150°C. If required, the reaction can be conducted under pressure.

Should the copolymers obtained from step (II) still comprise residues of acid anions, it may be preferable to remove these acid anions from the copolymer with the aid of an ion exchanger and preferably exchange them for hydroxide ions or carboxylate ions, more preferably hydroxide ions. This is the case especially when the acid anions present in the copolymer are halides or contain sulfur or nitrogen.

The copolymer obtained from reaction step (II) generally has a weight-average molecular weight Mw of 0.5 to 20 kDa, preferably 0.6 to 15, more preferably 0.7 to 7, even more preferably 1 to 7 and especially 1.5 to 4 kDa (determined by gel permeation chromatography with tetrahydrofuran and polystyrene as standard).

The number-average molecular weight Mn is usually from 0.5 to 10 kDa, preferably 0.6 to 5, more preferably 0.7 to 4, even more preferably 0.8 to 3 and especially 1 to 2 kDa (determined by gel permeation chromatography with tetrahydrofuran and polystyrene as standard).

The polydispersity is generally from 1 to 10, preferably from 1.1 to 8, more preferably from 1.2 to 7, even more preferably from 1.3 to 5 and especially from 1.5 to 3.

The content of acid groups in the copolymer is preferably from 1 to 8 mmol/g of copolymer, more preferably from 2 to 7.5, even more preferably from 3 to 7 mmol/g of copolymer.

In a preferred embodiment, the copolymers comprise a high proportion of adjacent carboxylic acid groups, which is determined by a measurement of adjacency. For this purpose, a sample of the copolymer is heat-treated between two Teflon films at a temperature of 290°C for a period of 30 minutes and an FTIR spectrum is recorded at a bubble-free site. The IR spectrum of Teflon is subtracted from the spectra obtained, the layer thickness is determined and the content of cyclic anhydride is determined.

In a preferred embodiment, the adjacency is at least 10%, preferably at least 15%, more preferably at least 20%, even more preferably at least 25% and especially at least 30%.

The olefin-carboxylic acid copolymer (A) is applied in the form of the free acid, i.e. COOH groups are present, or in the form of the anhydride which may be an intramolecular anhydride or an intermolecular anhydride linking two dicarboxylic acid molecules together, preferably in the form of a free acid. To a minor extent, some of the carboxylic functions may be present in salt form, e.g. as alkali or alkaline metal salts salts or as ammonium or substituted ammonium salts, depending on the pH value of the liquid phase. Preferably at least 50 % of all carboxylic acid groups are available in the form of the free acid as COOH-groups, more preferably at least 66 %, very preferably at least 75 %, even more preferably at least 85 %, and especially at least 95%. A single olefin-carboxylic acid copolymer (A) or a mixture of different olefin-carboxylic acid copolymers (A) may be used.

### Carrier oils

Carrier oils additionally used may be of mineral or synthetic nature. Suitable mineral carrier oils are fractions obtained in crude oil processing, such as brightstock or base oils having viscosities, for example, from the SN 500 - 2000 class; but also aromatic hydrocarbons, paraffinic hydrocarbons and alkoxyalkanols. Likewise useful is a fraction which is obtained in the refining of mineral oil and is known as "hydrocrack oil" (vacuum distillate cut having a boiling range of from about 360 to 500°C, obtainable from natural mineral oil which has been catalytically hydrogenated under high pressure and isomerized and also deparaffinized). Likewise suitable are mixtures of the abovementioned mineral carrier oils.

Examples of suitable synthetic carrier oils are polyolefins (polyalphaolefins or polyinternalole-fins), (poly)esters, (poly)alkoxylates, polyethers, aliphatic polyetheramines, alkylphenol-started polyethers, alkylphenol-started polyetheramines and carboxylic esters of long-chain alkanols.

Examples of suitable polyolefins are olefin polymers having M_{n =} 400 to 1800, in particular based on polybutene or polyisobutene (hydrogenated or unhydrogenated).

Examples of suitable polyethers or polyetheramines are preferably compounds comprising polyoxy-C₂- to C₄-alkylene moieties obtainable by reacting C₂- to C₆₀-alkanols, C₆- to C₃₀-alkanediols, mono- or di-C₂- to C₃₀-alkylamines, C₁- to C₃₀-alkylcyclohexanols or C₁- to C₃₀-alkylphenols with 1 to 30 mol of ethylene oxide and/or propylene oxide and/or butylene oxide per hydroxyl group or amino group, and, in the case of the polyetheramines, by subsequent reductive amination with ammonia, monoamines or polyamines. Such products are described more particularly in EP-A 310 875, EP-A 356 725, EP-A 700 985 and US-A 4,877,416. For example, the polyetheramines used may be poly-C₂- to C₆-alkylene oxide amines or functional derivatives thereof. Typical examples thereof are tridecanol butoxylates or isotridecanol butoxylates, heptadecanol butoxylates or isoheptadecanol butoxylates, tridecanol propoxylates or isotridecanol propoxylates, heptadecanol propoxylates or isoheptadecanol propoxylates, isononylphenol butoxylates and also polyisobutenol butoxylates and propoxylates, and also the corresponding reaction products with ammonia.

Examples of carboxylic esters of long-chain alkanols are more particularly esters of mono-, di- or tricarboxylic acids with long-chain alkanols or polyols, as described more particularly in DE-A 38 38 918. The mono-, di- or tricarboxylic acids used may be aliphatic or aromatic acids; particularly suitable ester alcohols or ester polyols are long-chain representatives having, for example, 6 to 24 carbon atoms. Typical representatives of the esters are adipates, phthalates, isophthalates, terephthalates and trimellitates of isooctanol, isononanol, isodecanol and isotridecanol, for example di(n- or isotridecyl) phthalate.

Further suitable carrier oil systems are described, for example, in DE-A 38 26 608, DE-A 41 42 241, DE-A 43 09 074, EP-A 452 328 and EP-A 548 617.

Examples of particularly suitable synthetic carrier oils are alcohol-started polyethers having about 5 to 35, preferably about 5 to 30, more preferably 10 to 30 and especially 15 to 30 C₃- to C₆-alkylene oxide units, for example propylene oxide, n-butylene oxide and isobutylene oxide units, or mixtures thereof, per alcohol molecule. Nonlimiting examples of suitable starter alcohols are long-chain alkanols or phenols substituted by long-chain alkyl in which the long-chain alkyl radical is especially a straight-chain or branched C₆- to C₁₈-alkyl radical. Particular examples include tridecanol, heptadecanol and nonylphenol. Particularly preferred alcohol-started polyethers are the reaction products (polyetherification products) of monohydric aliphatic C₆- to C₁₈-alcohols with C₃- to C₆-alkylene oxides. Examples of monohydric aliphatic C₆-C₁₈-alcohols are hexanol, heptanol, octanol, 2-ethylhexanol, nonyl alcohol, decanol, 3-propylheptanol, undecanol, dodecanol, tridecanol, tetradecanol, pentadecanol, hexadecanol, heptadecanol, octadecanol and the constitutional and positional isomers thereof. The alcohols can be used either in the form of the pure isomers or in the form of technical grade mixtures. A particularly preferred alcohol is tridecanol. Examples of C₃- to C₆-alkylene oxides are propylene oxide, such as 1,2-propylene oxide, butylene oxide, such as 1,2-butylene oxide, 2,3-butylene oxide, isobutylene oxide or tetrahydrofuran, pentylene oxide and hexylene oxide. Particular preference among these is given to C₃- to C₄-alkylene oxides, i.e. propylene oxide such as 1,2-propylene oxide and butylene oxide such as 1,2-butylene oxide, 2,3-butylene oxide and isobutylene oxide. Especially butylene oxide is used and mixtures of butylene oxide and propylene oxide.

Further suitable synthetic carrier oils are alkoxylated alkylphenols, as described in DE-A 10 102 913.

Particular carrier oils are synthetic carrier oils, particular preference being given to the above-described alcohol-started polyethers.

### Other additives

Typical other additives in the additive packages or fuels according to the invention may be friction modifier, dehazers, antioxidants, metal deactivators, and solvents for the packages.

### Friction modifier

Suitable friction modifiers are based typically on fatty acids, fatty acid esters or fatty acid amides. Typical examples are tall oil fatty acid, as described, for example, in WO 98/004656, and glyceryl monooleate. The reaction products, described in US 6743266 B2, of natural or synthetic oils, for example triglycerides, and alkanolamines are also suitable as such friction modifier.

Preferred lubricity improvers are described in WO 15/059063 and WO 10/005720. Furthermore, hydroxyl group-substituted tertiary amines as disclosed in WO 2014/23853 are preferred as friction modifiers.

### Dehazer

Suitable dehazer are, for example, the alkali metal or alkaline earth metal salts of alkylsubstituted phenol- and naphthalenesulfonates and the alkali metal or alkaline earth metal salts of fatty acids, and also neutral compounds such as alcohol alkoxylates, e.g. alcohol ethoxylates, phenol alkoxylates, e.g. tert-butylphenol ethoxylate or tert-pentylphenol ethoxylate, fatty acids, alkylphenols, condensation products of ethylene oxide (EO) and propylene oxide (PO), for example including in the form of EO/PO block copolymers, polyethyleneimines or else polysiloxanes.

Further suitable dehazers are EO/PO-based alkoxylates of alkylphenol-formaldehyde condensates (Novolac, resol or calixarene type), EO/PO-based alkoxylates of diols (e.g. propandiol, ethylene glycole), triols (e.g. glycerol or trimethylolpropane), ethylene diamine, or polyethylene-imine. Further suitable dehazers are alkybenzene sulfonic acids, dialkylsulfosuccinates or alkali metal or ammonium salts thereof. Suitable dehazers are described in WO 96/22343. Further suitable dehazers based on diglycidyl ethers are described in US 3383326 and US 3511882.

Other suitable dehazers are, for example, alkoxylated phenol-formaldehyde condensates, for example the products available under the trade names NALCO 7D07 (Nalco) and TOLAD 2683 (Petrolite).

### Antioxidants

Suitable antioxidants are, for example, substituted phenols, such as 2,6-di-tert-butylphenol, 2,6-di-tert-butyl-4-methyl phenol, 2,4-di-tert-butyl-6-methylphenol, preferably hindered phenols with an ester group bearing radical in para position, such as 3-[3,5-bis-(dimethylethyl)-4-hydroxyphenyl] propanoic acid C₆- to C₂₀-alkyl esters, e.g. 2-ethylhexyl- or stearylester, and also phenylenediamines such as N,N'-di-sec-butyl-p-phenylenediamine.

### Metal deactivators

Suitable metal deactivators are, for example, salicylic acid derivatives such as N,N'-disalicylidene-1,2-propanediamine.

### Solvents

Suitable solvents are, for example, nonpolar organic solvents such as aromatic and aliphatic hydrocarbons, for example toluene, xylenes, white spirit and products sold under the trade names SHELLSOL (Royal Dutch/Shell Group) and EXXSOL (ExxonMobil), and also polar organic solvents, for example, alcohols such as 2-ethylhexanol, 2-propylheptanol, decanol, isotridecanol and isoheptadecanol. Such solvents are usually added to the fuel together with the aforementioned additives and coadditives, which they are intended to dissolve or dilute for better handling.

### Fuels

In the context of the present invention, gasoline fuels mean liquid hydrocarbon distillate fuels boiling in the gasoline range. It is in principle suitable for use in all types of gasoline, including "light" and "severe" gasoline species. The gasoline fuels may also contain amounts of other fuels such as, for example, ethanol.

Typically, gasoline fuels, which may be used according to the present invention exhibit, in addition, one or more of the following features:
The aromatics content of the gasoline fuel is preferably not more than 50 volume % and more preferably not more than 35 volume %. Preferred ranges for the aromatics content are from 1 to 45 volume % and particularly from 5 to 35 volume %.

The sulfur content of the gasoline fuel is preferably not more than 100 ppm by weight and more preferably not more than 10 ppm by weight. Preferred ranges for the sulfur content are from 0.5 to 150 ppm by weight and particularly from 1 to 10 ppm by weight.

The gasoline fuel has an olefin content of not more than 21 volume %, preferably not more than 18 volume %, and more preferably not more than 10 volume %. Preferred ranges for the olefin content are from 0.1 to 21 volume % and particularly from 2 to 18 volume %.

The gasoline fuel has a benzene content of not more than 1.0 volume % and preferably not more than 0.9 volume %. Preferred ranges for the benzene content are from 0 to 1.0 volume % and preferably from 0.05 to 0.9 volume %.

The gasoline fuel has an oxygen content of not more than 45 weight %, preferably from 0 to 45 weight %, and most preferably from 0.1 to 3.7 weight % (first type) or most preferably from 3.7 to 45 weight % (second type). The gasoline fuel of the second type mentioned above is a mixture of lower alcohols such as methanol or especially ethanol, which derive preferably from natural source like plants, with mineral oil based gasoline, i.e. usual gasoline produced from crude oil. An example for such gasoline is "E 85", a mixture of 85 volume % of ethanol with 15 volume % of mineral oil based gasoline. Also a fuel containing 100 % of a lower alcohol, especially ethanol, is suitable.

The amount of alcohols and ethers contained in the gasoline may vary over wide ranges. Typical maximum contents are e.g. methanol 15% by volume, ethanol 85% by volume, isopropanol 20% by volume, tert-butanol 15% by volume, isobutanol 20% by volume and ethers containing 5 or more carbon atoms in the molecule 30% by volume.

The summer vapor pressure of the gasoline fuel is usually not more than 70 kPa and preferably not more than 60 kPa (at 37°C).

The research octane number ("RON") of the gasoline fuel is usually from 90 to 100. A usual range for the corresponding motor octane number ("MON") is from 80 to 90.

The above characteristics are determined by conventional methods (DIN EN 228).

As pointed out above, the gasoline fuels according to the present invention comprise at least one amide of formula (I) in an amount of from 25 to 1000 ppm, preferably from 50 to 500 ppm, more preferably from 75 to 250 ppm.

The deposit control agent or mixture of a plurality of such additives is present in the gasoline fuels in the case of polyalkenemono- or polyalkenepolyamines or Mannich adducts typically in an amount of from 10 to 1000 ppm by weight, preferably of from 25 to 500 ppm by weight, more preferably of from 50 to 250 ppm by weight.

In the case of quaternary ammonium compounds as deposit control agents they are typically present in the gasoline fuels in an amount of from 10 to 100 ppm by weight, preferably of from 20 to 50 ppm by weight,

The one or more corrosion inhibitors, if any, are present in the gasoline fuels normally in an amount of from 0.1 to 10 ppm by weight, preferably of from 0.2 to 8 ppm by weight, more preferably of from 0.3 to 7 ppm by weight, most preferably of from 0.5 to 5 ppm by weight, for example of from 1 to 3 ppm by weight.

The one or more carrier oils, if any, are present in the gasoline fuels normally in an amount of form 10 to 3.000 ppm by weight, preferably of from 20 to 1000 ppm by weight, more preferably of from 50 to 700 ppm by weight, most preferably of from 70 to 500 ppm by weight.

One or more dehazers as additive component, if any, are present in the gasoline fuels generally in an amount of from 0.5 to 100 ppm by weight, preferably of from 1 to 50 ppm by weight, more preferably of from 1.5 to 40 ppm by weight, most preferably of from 2 to 30 ppm by weight, for example of from 3 to 20 ppm by weight.

The other additive components described above each, if any, are present in the gasoline fuels generally in an amount of from 0.5 to 200 ppm by weight, preferably of from 1 to 100 ppm by weight, more preferably of from 1.5 to 40 ppm by weight, most preferably of from 2 to 30 ppm by weight.

Subject matter of the present invention is also a fuel additive concentrate suitable for use in gasoline fuels comprising
0.01 to 40% by weight, preferably 0.05 to 35% by weight, more preferably 1 to 30% by weight, even more preferably 5 to 25 % by weight of the at least one amide of formula (I);
10 to 70% by weight, preferably 15 to 60% by weight, more preferably 20 to 50% by weight, of the at least one deposit control agent;
0.25 to 5% by weight, preferably 0.5 to 5 by weight, more preferably 0.75 to 3.5% by weight, most preferably 1.0 to 2% by weight, of at least one corrosion inhibitor;
0 to 80% by weight, preferably 5 to 60% by weight, more preferably 10 to 40% by weight, of at least one carrier oil;
0 to 80% by weight, preferably 5 to 50% by weight, more preferably 10 to 40% by weight, of at least one solvent or diluent; and
0 to 15% by weight, preferably 0.5 to 10 by weight, more preferably 1 to 8% by weight, most preferably 3 to 7% by weight, of each of the other additive components described above, if any;
with the proviso that the sum of components always results in 100%.

The amounts given throughout the text refer to the pure components excluding e.g. solvent, unless stated otherwise.

### Examples

### Synthesis Example 1

A mixture of isononanoic acid (0.95 eq.) and KEROCOM PIBA 03 (1 eq., CAS No. 886464-29-5, commercially available from BASF SE, Ludwigshafen, with an amine number of 19 mg KOH/g, 65 wt% solution in Mihagol) was heated between 195 and 220 °C under an N₂ atmosphere for 9 h using a Dean-Stark apparatus and yielding an amide-containing reaction mixture with an amine number of 2.4 mg KOH/g and an acid number of 1.9 mg KOH/g. The mixture was used in application tests without further purification.

### Synthesis Example 2

A mixture of carboxylic acids from coconut oil (0.95 eq., KLK Oleo Palmera B1209, mixture of C₈-, C₁₀-, C₁₂-, C₁₄-, C₁₆-, and C₁₈-fatty acids with lauric acid being the main constituent) and KEROCOM PIBA 03 (1 eq., CAS No. 886464-29-5, commercially available from BASF SE, Ludwigshafen, with an amine number of 21 mg KOH/g, 65 wt% solution in Mihagol) was heated between 195 and 220 °C under an N₂ atmosphere for 5.5 h using a Dean-Stark apparatus. Another 0.08 eq. of the carboxylic acid were added and heating was continued for 4 h at 220 °C yielding an amide-containing reaction mixture with an amine number of 2.4 mg KOH/g and an acid number of 1.0 mg KOH/g. The mixture was used in application tests without further purification.

### Synthesis Example 3

A mixture of carboxylic acids from coconut oil (1.014 eq., KLK Oleo Palmera B1209, mixture of C₈-, C₁₀-, C₁₂-, C₁₄-, C₁₆-, and C₁₈-fatty acids with lauric acid being the main constituent) and KEROCOM PIBA 03 (1 eq., CAS No. 886464-29-5, commercially available from BASF SE, Ludwigshafen, with an amine number of 20 mg KOH/g, 65 wt% solution in Mihagol) was heated between 140 and 170 °C under an N₂ atmosphere for 6 h using a Dean-Stark apparatus. Then the pressure was reduced to 300 mbar and heating was continued for 7 h at 170 °C yielding an amide-containing reaction mixture with an amine number of 3.2 mg KOH/g and an acid number of 1.9 mg KOH/g. The mixture was used in application tests without further purification.

### Synthesis Example 4

The amide of the reaction of isostearic acid and n-butyl amine according to US 7846224 B2 was conducted as follows:
293 g isostearic chlorid (1 eq., TCI) were added to a mixture of 71 g butylamine (1. eq.) and 118 g triethylamine (1.21 eq.) in 1450 mL dichloromethane at room temperature. The reaction mixture was stirred for 24 h. After an aqueous work-up the reaction product was diluted in 2-ethylhexanol resulting in a solution containing 20 w% of the desired isostearoyl butylamide with an amine number of 0.1 mg KOH/g and an acid number of 5.2 mg KOH/g. The mixture was used in application tests without further purification.

### Application Example 1 - Fuel Economy

The amide of Synthesis Example 1 was used in approx. 65 wt% solution in a base fuel (US RUL, E10, LAC) and submitted to a fuel economy test using the ECE cycle with a Ford Escape. In the morning the test was run using the base fuel and compared with the additised fuel in the afternoon.

As Comparison 1 a friction modifier according to WO 2010/005720 A1, Example 2 (neat) was used.

| Additive | Amount [mg/kg] | Change in Fuel Economy [%] |
|---|---|---|
| None | -- | -0.16 |
| Comparison 1 | 75 | 0.27 |
| Synthesis Example 1 | 115 | 1.83 |

A positive value means savings in fuel consumption.

The value of -0.16 (entry 1) represents the repeatability and reproducibility of this fuel economy test.

### Application Example 2 - Fuel Economy

An amide of lauric acid and PIBA was obtained in an analogous matter to Synthesis Example 2 was used in approx. 65 wt% solution and submitted to a fuel economy test as in Application Example 1.

As Comparison 2 a friction modifier according to WO 2015/059063 A1, Example 3, made of C₈-C₁₀-monocarboxylic acid, adipic acid, and trimethylolpropane (neat) was used.

| Additive | Amount [mg/kg] | Change in Fuel Economy [%] (repeat) |
|---|---|---|
| None | -- | 0.29 |
| Comparison 2 | 90 | 0.89 |
| Lauric acid amide | 140 | 1.28 |

### Application Example 3 - Deposits (Keep Clean)

An engine test was conducted over 60 hours according to CEC F-020-98 (keep-clean mode) with MIRO 95-octane E10 fuel and in a port fuel injector engine (M111E) and the deposits on the intake valves (intake valve deposits, IVD, average mg per valve) were determined.

Formulations used and the results are listed in the following table:

| Entry | Amide | Deposit Control Agent* | Carrier Oil** | Quat*** | IVD |
|---|---|---|---|---|---|
| 1 | 0 | 0 | 0 | 0 | 147 |
| 2 | 0 | 154 | 36 | 25 | 13 |
| 3 | 0 | 175 | 41 | 25 | 5 |
| 4 | 175**** | 0 | 41 | 25 | 48 |
| 5 | 115***** | 150 | 35 | 25 | 0 |
| 6 | 115***** | 130 | 30 | 25 | 2 |
| 7 | 115***** | 110 | 26 | 25 | 19 |
| 8 | 115****** | 110 | 124 | 25 | 0 |
| 9 | 115****** | 110 | 101 | 25 | 0 |
| 10 | 115****** | 110 | 79 | 25 | 1 |

| | | | | | |
|---|---|---|---|---|---|
| * Deposit Control Additive: Kerocom(R) PIBA (65% by weight solution of polyisobutylene amine based on high-reactivity poly-isobutene (after hydroformylation and amination), Mn=1000, in an aliphatic hydrocarbon mixture) ** Carrier Oil: propoxylated tridecanol derived from trimerbutene (after hydroformylation and hydrogenation) *** Quat: Quaternary ammonium compound preparatory example 6 from WO 2014/195464. **** Amide obtained in analogous matter according to Synthesis Example 1 ***** Amide obtained in analogous matter according to Synthesis Example 1, 65 wt% solution ****** Amide obtained in analogous matter according to Synthesis Example 1 as 67 wt% solution | | | | | |

A comparison of Entries 2 and 5 shows that the use of the amides according to the invention additionally to polyisobutene amine drastically reduces IVD.

### Application Example 4 - Deposits (Clean Up)

An engine test was conducted according to CEC F-020-98 (clean up-mode) with MIRO 95-octane E10 fuel and in a port fuel injector engine (M111E):
In the dirty-up-clean-up sequence dirty-up is achieved by running the engine over 60 hours with 96 vol% EN 228-compliant E10 base fuel + 4 vol% of a fuel prone to forming intake valve deposits. The relative change of IVD (intake valve deposits, IVD, average mg per valve) is determined as described above. The subsequent clean-up run is done with 100 vol% EN 228-compliant E10 additized base fuel over 60 h and the deposits on the intake valves were determined.

Formulations used and the results are listed in the following table:

| Entry | Amide****** | Deposit Control Agent* | Carrier Oil** | IVD | Clean Up [%] |
|---|---|---|---|---|---|
| 1 | 0 | 537 | 297 | 195 → 29 | 85 |
| 2 | 345 | 330 | 371 | 201 → 45 | 78 |
| 3 | 345 | 330 | 236 | 185 → 38 | 79 |

| | | | | | |
|---|---|---|---|---|---|
| * Deposit Control Additive: Kerocom(R) PIBA (65% by weight solution of polyisobutylene amine based on high-reactivity poly-isobutene (after hydroformylation and amination), Mn=1000, in an aliphatic hydrocarbon mixture) ** Carrier Oil: propoxylated tridecanol derived from trimerbutene (after hydroformylation and hydrogenation) ****** Amide obtained in analogous matter according to Synthesis Example 1 as 67 wt% solution | | | | | |

It can be seen that the use of the amides according to the invention additionally to polyisobutene amine allows for a similar clean up-performance while the amount of polyisobutene amine can be reduced.

### Application Example 5 - Keep Clean

An engine test was conducted as described in Application Example 3 with a different MIRO 95-octane E10 fuel and the deposits on the intake valves (intake valve deposits, IVD, average mg per valve) were determined.

Formulations used and the results are listed in the following table:

| Entry | Amide | Deposit Control Agent* | Carrier Oil** | Corrosion Inhibitor*** | IVD |
|---|---|---|---|---|---|
| 1 | 0 | 0 | 0 | 0 | 179 |
| 2 | 115**** | 110 | 79 | 4 | 15 |
| 3 | 75***** | 110 | 79 | 4 | 157 |
| 4 | 375****** | 110 | 79 | 4 | 75 |

| | | | | | |
|---|---|---|---|---|---|
| * Deposit Control Additive: Kerocom(R) PIBA (65% by weight solution of polyisobutylene amine based on high-reactivity poly-isobutene (after hydroformylation and amination), Mn=1000, in an aliphatic hydrocarbon mixture) ** Carrier Oil: propoxylated tridecanol derived from trimerbutene (after hydroformylation and hydrogenation) *** Corrosion Inhibitor: Hydrolised copolymer of maleic anhydride and C20- to C24-olefins as described in WO 15/113681, Example 2. **** Amide obtained in analogous matter according to Synthesis Example 2 ***** Comparison 1 as described in Application Example 1 ****** Synthesis Example 4, 20 wt% solution | | | | | |

### Application Example 6 (Leaching Tests)

In a closed 250 ml glass bottle copper (49 _{×} 25 _{×} 1.5 mm) and lead platelets (50 _{×} 20 _{×} 1 mm) were placed in 200 ml of the fuel comprising the additives given in the tables below at ambient temperature for 8 weeks and the metal content of the fuel was determined using atom absorption spectrometry. Before use the platelets were brushed and wiped with xylene and acetone and were hung into the glass bottles so that they are completely covered with fuel.

### Fuel: E0 base fuel

**Table 6a**

| Additive and Amount | Start | 2 Weeks | 8 Weeks |
|---|---|---|---|
| None | < 1 ; < 1 | < 1 ; < 1 | < 1 ; < 1 |
| 33 mg/kg Oleic Acid | < 1 ; < 1 | < 1 ; 13 | < 1 ; 24 |
| 150 mg/kg Comparison 1 as described in Application Example 1 | < 1 ; < 1 | < 1 ; < 1 | 1 ; 4 |
| 351 mg/kg Comparison 2 as described in Application Example 2 | < 1 ; < 1 | < 1 ; < 1 | < 1 ; 1 |
| 345 mg/kg of Amide * | < 1 ; < 1 | < 1 ; < 1 | < 1 ; < 1 |
| 1345 mg/kg of Amide ** | < 1 ; < 1 | < 1 ; < 1 | < 1 ; < 1 |

| | | | |
|---|---|---|---|
| Left value: copper content, right value: lead content [mg/kg] * Amide obtained in analogous matter according to Synthesis Example 1 ** Amide obtained in analogous matter according to Synthesis Example 2 | | | |

### Fuel: E10 base fuel

**Table 6b**

| Additive and Amount | Start | 2 Weeks | 8 Weeks |
|---|---|---|---|
| None | < 1 ; < 1 | < 1 ; < 1 | 1 ; 5 |
| 33 mg/kg Oleic Acid | < 1 ; < 1 | 9 ; 4 | 16 ; 6 |
| 150 mg/kg Comparison 1as described in Application Example 1 | < 1 ; < 1 | 5 ; 1 | 9 ; 4 |
| 351 mg/kg Comparison 2 as described in Application Example 2 | < 1 ; < 1 | 1 ; 1 | 2 ; 4 |
| 345 mg/kg of Amide * | < 1 ; < 1 | 4 ; < 1 | 7 ; 3 |
| 345 mg/kg of Amide ** | < 1 ; < 1 | 4 ; < 1 | 6 ; 6 |

| | | | |
|---|---|---|---|
| Left value: copper content, right value: lead content [mg/kg] * Amide obtained in analogous matter according to Synthesis Example 1 ** Amide obtained in analogous matter according to Synthesis Example 2 | | | |

It can easily be seen that the amides according to the invention exhibit less leaching of metal ions than the chemically related friction modifier Comparison 1, which also comprises an amide group. In comparison with the ester group friction modifier of Comparison 2 the leaching of metal ions depends on the fuel and the metal ion: In E0 fuels the amides according to the invention are advantageous, in fuels comprising alkanols the amides according to the invention are advantageous with regard to leaching of lead. Leaching of copper is within the accuracy of the measurement comparable with Comparison 2 but still on an acceptable level.

## Claims

1. Gasoline Fuel, comprising at least one amide of formula (I)
R¹-(C=O)-(NR²)-R³
wherein
R¹ is a linear or branched, preferably linear C₇- to C₂₉-alkyl, or C₇- to C₂₉-alkenyl, preferably C₇- to C₂₃-alkyl or C₇- to C₂₃-alkenyl
R² is hydrogen or C₁- to C₄-alkyl, and
R³ is a hydrocarbyl residue comprising 12 to 200 carbon atoms obtainable from polymerisation of an olefin.

2. Gasoline Fuel according to Claim 1, **characterised in that** R² is hydrogen.

3. Gasoline Fuel according to Claim 1 or 2, **characterised in that** R¹ is a branched alkyl residue.

4. Gasoline Fuel according to any of the preceding claims, **characterised in that** the corresponding carboxylic acid R¹-COOH is selected from the group consisting of octanoic acid (caprylic acid), 2-ethylhexanoic acid, 3,5,5-trimethyl hexanoic acid, nonanoic acid, isononanoic acid, 2-propylheptanoic acid, decanoic acid (capric acid), undecanoic acid, dodecanoic acid (lauric acid), tridecanoic acid, tetradecanoic acid (myristic acid), hexadecanoic acid (palmitic acid), heptadecanoic acid, octadecanoic acid (stearic acid), isostearic acid, oleic acid, linoleic acid, linolaidic acid, erucic acid, arachidic acid, behenic acid, lignoceric acid, and cerotic acid.

5. Gasoline Fuel according to any of the preceding claims, **characterised in that** the corresponding carboxylic acid R¹-COOH is isononanoic acid.

6. Gasoline Fuel according to any of Claims 1 or 2, **characterised in that** the corresponding carboxylic acid R¹-COOH is selected from the group consisting of caprylic acid, capric acid, lauric acid, myristic acid, palmitic acid, oleic acid, stearic acid, elaidic acid, and linoleic acid or mixtures thereof.

7. Gasoline Fuel according to any of the preceding claims, **characterised in that** in the corresponding amine R³-NHR² in which the residue R² is hydrogen is obtainable by polymerisation of an olefin selected from the group consisting of propene, 1-butene, and iso-butene, followed by hydroformylation and reductive amination, preferably with ammonia.

8. Gasoline Fuel according to any of the Claims 1 to 6, **characterised in that** in the corresponding amine R³-NHR² the residue R² is hydrogen and the residue R³ is derived from a polyisobutene with the weight average molecular weight of from 168 to 2300, more preferably of from 224 to 1500, even more preferably of from 550 to 1300, most preferably of from 700 to 1300, and especially of 950 to 1050.

9. Gasoline Fuel according to any of the preceding claims, further comprising at least one deposit control agent selected from the group consisting of
- quaternary ammonium compounds,
- Mannich adducts, and
- polyalkenemono- or polyalkenepolyamines having a number average molecular weight in the range 300 to 5000.

10. Gasoline Fuel according to any of the preceding claims, further comprising at least one carrier oil, preferably comprising polyoxy-C₂- to C₄-alkylene moieties obtainable by reacting C₂-to C₆₀-alkanols, C₆- to C₃₀-alkanediols, mono- or di-C₂- to C₃₀-alkylamines, C₁- to C₃₀-alkylcyclohexanols or C₁- to C₃₀-alkylphenols with 1 to 30 mol of ethylene oxide and/or propylene oxide and/or butylene oxide per hydroxyl group or amino group, and, in the case of the polyetheramines, by subsequent reductive amination with ammonia, monoamines or polyamines.

11. Fuel additive packages, comprising at least one amide of formula (I) according to any of the preceding claims, further comprising at least one deposit control agent selected from the group consisting of
- quaternary ammonium compounds,
- Mannich adducts, and
- polyalkenemono- or polyalkenepolyamines having a number average molecular weight in the range 300 to 5000.

12. Fuel additive packages according to Claim 11, further comprising at least one carrier oil, preferably comprising polyoxy-C₂- to C₄-alkylene moieties obtainable by reacting C₂- to C₆₀-alkanols, C₆- to C₃₀-alkanediols, mono- or di-C₂- to C₃₀-alkylamines, C₁- to C₃₀-alkylcyclohexanols or C₁- to C₃₀-alkylphenols with 1 to 30 mol of ethylene oxide and/or propylene oxide and/or butylene oxide per hydroxyl group or amino group, and, in the case of the polyetheramines, by subsequent reductive amination with ammonia, monoamines or polyamines.

13. Use of an amide of formula (I) according to any of the Claims 1 to 8 as an additive in a gasoline fuel.

14. Use according to Claim 13 for improving the fuel efficiency and/or as a fuel economy additive.

15. Use according to Claim 13 for reducing or removing deposits on the intake valves and/or reducing or removing the fouling of injector nozzles, especially in direct injection spark ignition engines.

## Patentansprüche

1. Ottokraftstoff, umfassend mindestens ein Amid der Formel (I)
R¹-(C=O)-(NR²)-R³
wobei
R¹ für ein lineares oder verzweigtes, vorzugsweise lineares, C₇- bis C₂₉-Alkyl oder C₇- bis C₂₉-Alkenyl, vorzugsweise C₇- bis C₂₃-Alkyl oder C₇- bis C₂₃-Alkenyl, steht,
R² für Wasserstoff oder C₁- bis C₄-Alkyl steht und
R³ für einen Hydrocarbylrest mit 12 bis 200 Kohlenstoffatomen steht, der aus der Polymerisation eines Olefins erhältlich ist.

2. Ottokraftstoff nach Anspruch 1, **dadurch gekennzeichnet, dass** R² für Wasserstoff steht.

3. Ottokraftstoff nach Anspruch 1 oder 2, **dadurch gekennzeichnet, dass** R¹ für einen verzweigten Alkylrest steht.

4. Ottokraftstoff nach einem der vorhergehenden Ansprüche, **dadurch gekennzeichnet, dass** die entsprechende Carbonsäure R¹-COOH aus der Gruppe bestehend aus Octansäure (Caprylsäure), 2-Ethylhexansäure, 3,5,5-Trimethylhexansäure, Nonansäure, Isononansäure, 2-Propylheptansäure, Decansäure (Caprinsäure), Undecansäure, Dodecansäure (Laurinsäure), Tridecansäure, Tetradecansäure (Myristinsäure), Hexadecansäure (Palmitinsäure), Heptadecansäure, Octadecansäure (Stearinsäure), Isostearinsäure, Ölsäure, Linolsäure, Linolaidinsäure, Erucasäure, Arachidinsäure, Behensäure, Lignocerinsäure und Cerotinsäure ausgewählt ist.

5. Ottokraftstoff nach einem der vorhergehenden Ansprüche, **dadurch gekennzeichnet, dass** es sich bei der entsprechenden Carbonsäure R¹-COOH um Isononansäure handelt.

6. Ottokraftstoff nach Anspruch 1 oder 2, **dadurch gekennzeichnet, dass** die entsprechende Carbonsäure R¹-COOH aus der Gruppe bestehend aus Caprylsäure, Caprinsäure, Laurinsäure, Myristinsäure, Palmitinsäure, Ölsäure, Stearinsäure, Elaidinsäure und Linolsäure und Mischungen davon ausgewählt ist.

7. Ottokraftstoff nach einem der vorhergehenden Ansprüche, **dadurch gekennzeichnet, dass** das entsprechende Amin R³-NHR², in dem der Rest R² für Wasserstoff steht, durch Polymerisation eines Olefins, das aus der Gruppe bestehend aus Propen, 1-Buten und Isobuten ausgewählt ist, und anschließende Hydroformylierung und reduktive Aminierung, vorzugsweise mit Ammoniak, erhältlich ist.

8. Ottokraftstoff nach einem der Ansprüche 1 bis 6, **dadurch gekennzeichnet, dass** in dem entsprechenden Amin R³-NHR² der Rest R² für Wasserstoff steht und der Rest R³ von einem Polyisobuten mit einem gewichtsmittleren Molekulargewicht von 168 bis 2300, weiter bevorzugt von 224 bis 1500, noch weiter bevorzugt von 550 bis 1300, ganz besonders bevorzugt von 700 bis 1300 und speziell von 950 bis 1050 abgeleitet ist.

9. Ottokraftstoff nach einem der vorhergehenden Ansprüche, ferner umfassend mindestens ein Mittel zur Verhinderung von Ablagerungen, ausgewählt aus der Gruppe bestehend aus
- quartären Ammoniumsverbindungen,
- Mannich-Addukten und
- Polyalkenmono- oder Polyalkenpolyaminen mit einem zahlenmittleren Molekulargewicht im Bereich von 300 bis 5000.

10. Ottokraftstoff nach einem der vorhergehenden Ansprüche, ferner umfassend mindestens ein vorzugsweise Polyoxy-C₂- bis -C₄-alkylengruppierungen enthaltendes Trägeröl, das durch Umsetzung von C₂- bis C₆₀-Alkanolen, C₆- bis C₃₀-Alkandiolen, Mono- oder Di-C₂- bis C₃₀-alkylaminen, C₁- bis C₃₀-Alkylcyclohexanolen oder C₁- bis C₃₀-Alkylphenolen mit 1 bis 30 mol Ethylenoxid und/oder Propylenoxid und/oder Butylenoxid pro Hydroxylgruppe oder Aminogruppe und, im Fall der Polyetheramine, durch anschließende reduktive Aminierung mit Ammoniak, Monoaminen oder Polyaminen erhältlich ist.

11. Kraftstoffadditivpakete, umfassend mindestens ein Amid der Formel (I) nach einem der vorhergehenden Ansprüche, ferner umfassend mindestens ein Mittel zur Verhinderung von Ablagerungen, ausgewählt aus der Gruppe bestehend aus
- quartären Ammoniumsverbindungen,
- Mannich-Addukten und
- Polyalkenmono- oder Polyalkenpolyaminen mit einem zahlenmittleren Molekulargewicht im Bereich von 300 bis 5000.

12. Kraftstoffadditivpakete nach Anspruch 11, ferner umfassend mindestens ein vorzugsweise Polyoxy-C₂- bis - C₄-alkylengruppierungen enthaltendes Trägeröl, das durch Umsetzung von C₂- bis C₆₀-Alkanolen, C₆- bis C₃₀-Alkandiolen, Mono- oder Di-C₂- bis C₃₀-alkylaminen, C₁-bis C₃₀-Alkylcyclohexanolen oder C₁- bis C₃₀-Alkylphenolen mit 1 bis 30 mol Ethylenoxid und/oder Propylenoxid und/oder Butylenoxid pro Hydroxylgruppe oder Aminogruppe und, im Fall der Polyetheramine, durch anschließende reduktive Aminierung mit Ammoniak, Monoaminen oder Polyaminen erhältlich ist.

13. Verwendung eines Amids der Formel (I) nach einem der Ansprüche 1 bis 8 als Additiv in einem Ottokraftstoff.

14. Verwendung nach Anspruch 13 zur Verbesserung der Kraftstoffeffizienz und/oder als Kraftstoffökonomieadditiv.

15. Verwendung nach Anspruch 13 zur Verringerung oder Entfernung von Ablagerungen in den Einlassventilen und/oder zur Verringerung oder Entfernung der Verschmutzung von Einspritzdüsen, insbesondere in direkteinspritzenden Ottomotoren.

## Revendications

1. Carburant de type essence comprenant au moins un amide de formule (I)
R¹- (C=O) - (NR²) -R³
dans lequel
R¹ est un alkyle en C₇ à C₂₉ linéaire ou ramifié, de préférence linéaire, ou un alcényle en C₇ à C₂₉, de préférence un alkyle en C₇ à C₂₃ ou un alcényle en C₇ à C23
R² est hydrogène ou un alkyle en C₁ à C₄, et
R³ est un résidu hydrocarbyle comprenant 12 à 200 atomes de carbone pouvant être obtenu par polymérisation d'une oléfine.

2. Carburant de type essence selon la revendication 1, **caractérisé en ce que** R² est hydrogène.

3. Carburant de type essence selon la revendication 1 ou 2, **caractérisé en ce que** R¹ est un résidu alkyle ramifié.

4. Carburant de type essence selon l'une quelconque des revendications précédentes, **caractérisé en ce que** l'acide carboxylique R²-COOH correspondant est choisi dans le groupe constitué de l'acide octanoïque (acide caprylique), l'acide 2-éthylhexanoïque, l'acide 3,5,5-triméthylhexanoïque, l'acide nonanoïque, l'acide isononanoïque, l'acide 2-propylheptanoïque, l'acide décanoïque (acide caprique), l'acide undécanoïque, l'acide dodécanoïque (acide laurique), l'acide tridécanoïque, l'acide tétradécanoïque (acide myristique), l'acide hexadécanoïque (acide palmitique), l'acide heptadécanoïque, l'acide octadécanoïque (acide stéarique), l'acide isostéarique, l'acide oléique, l'acide linoléique, l'acide linolaïdique, l'acide érucique, l'acide arachidique, l'acide béhénique, l'acide lignocérique et l'acide cérotique.

5. Carburant de type essence selon l'une quelconque des revendications précédentes, **caractérisé en ce que** l'acide carboxylique R¹-COOH correspondant est l'acide isononanoïque.

6. Carburant de type essence selon l'une quelconque des revendications 1 ou 2, **caractérisé en ce que** l'acide carboxylique R¹-COOH correspondant est choisi dans le groupe constitué par l'acide caprylique, l'acide caprique, l'acide laurique, l'acide myristique, l'acide palmitique, l'acide oléique, l'acide stéarique, l'acide élaïdique et l'acide linoléique ou leurs mélanges.

7. Carburant de type essence selon l'une quelconque des revendications précédentes, **caractérisé en ce que** l'amine R³-NHR² correspondante dans laquelle le résidu R² est hydrogène peut être obtenue par polymérisation d'une oléfine choisie dans le groupe constitué par le propène, le 1-butène et l'isobutène, suivie d'une hydroformylation et d'une amination réductrice, de préférence avec de l'ammoniac.

8. Carburant de type essence selon l'une quelconque des revendications 1 à 6, **caractérisé en ce que** dans l'amine R³-NHR² correspondante, le résidu R² est hydrogène et le résidu R³ est dérivé d'un polyisobutène ayant un poids moléculaire moyen en poids de 168 à 2300, plus préférablement de 224 à 1500, encore plus préférablement de 550 à 1300, de manière préférée entre toutes de 700 à 1300, et en particulier de 950 à 1050.

9. Carburant de type essence selon l'une quelconque des revendications précédentes, comprenant en outre au moins un agent de contrôle de dépôt choisi dans le groupe constitué de :
- composés d'ammonium quaternaire,
- adduits de Mannich, et
- polyalcène(mono- ou polyalcène)polyamines ayant un poids moléculaire moyen en nombre dans la plage de 300 à 5000.

10. Carburant de type essence selon l'une quelconque des revendications précédentes, comprenant en outre au moins une huile support, comprenant de préférence des fragments polyoxy-(alkylène en C₂ à C₄) pouvant être obtenus par réaction d'alcanols en C₂ à C₆₀, d'alcanediols en C₆ à C₃₀, de mono- ou di-(alkyle en C₂ à C₃₀)amines, de (alkyle en C₁ à C₃₀) cyclohexanols ou de (alkyle en C₁ à C₃₀) phénols avec 1 à 30 moles d'oxyde d'éthylène et/ou d'oxyde de propylène et/ou d'oxyde de butylène par groupe hydroxyle ou groupe amino, et, dans le cas des polyétheramines, par amination réductrice ultérieure avec de l'ammoniac, des monoamines ou des polyamines.

11. Préformulation d'additifs pour carburant, comprenant au moins un amide de formule (I) selon l'une quelconque des revendications précédentes, comprenant en outre au moins un agent de contrôle de dépôt choisi dans le groupe constitué de
- composés d'ammonium quaternaire,
- adduits de Mannich, et
- polyalcène(mono- ou polyalcène)polyamines ayant un poids moléculaire moyen en nombre dans la plage de 300 à 5000 .

12. Préformulation d'additifs pour carburant selon la revendication 11, comprenant en outre au moins une huile support, comprenant de préférence des fragments polyoxy-(alkylène en C₂ à C₄) pouvant être obtenus par réaction d'alcanols en C₂ à C₆₀, d'alcanediols en C₆ à C₃₀, de mono- ou di-(alkyle en C₂ à C₃₀) amines, de (alkyle en C₁ à C₃₀)cyclohexanols ou de (alkyle en C₁ à C₃₀) phénols avec 1 à 30 moles d'oxyde d'éthylène et/ou d'oxyde de propylène et/ou d'oxyde de butylène par groupe hydroxyle ou groupe amino, et, dans le cas des polyétheramines, par amination réductrice ultérieure avec de l'ammoniac, des monoamines ou des polyamines.

13. Utilisation d'un amide de formule (I) selon l'une quelconque des revendications 1 à 8 comme additif dans un carburant de type essence.

14. Utilisation selon la revendication 13 pour améliorer le rendement énergétique du carburant et/ou comme additif d'économie de carburant.

15. Utilisation selon la revendication 13 pour réduire ou éliminer les dépôts sur les soupapes d'admission et/ou réduire ou éliminer l'encrassement des buses d'injecteur, notamment dans les moteurs à allumage par étincelle à injection directe.
